# EUROPEAN PATENT APPLICATION

(11) **EP 4 180 039 A1**
(43) Date of publication of application: **17.05.2023**
(21) Application number: 21838225.7
(22) Date of filing: 09.07.2021
(51) Int. Cl.: A61K 31/435, A61P 33/02, C07D 221/00, C07D 215/02

(54) **<SMALLCAPS/>? ? ?LEISHMANIA? ? ? ? ?USE OF SIMPLE ACYCLIC POLYAMINES FOR THE TREATMENT OF DISEASES CAUSED BY PARASITES OF THE GENUS**

(30) Priority: 10.07.2020 ES 202030713
(71) Applicant: Universidad de Granada, 18071 Granada (ES); Universitat de València, 46010 Valencia (ES)
(72) Inventor: GARGÍA-ESPAÑA MONSONÍS, Enrique, 46010 Valencia (ES); CLARES GARCÍA, María Paz, 46010 Valencia (ES); DELGADO PINAR, Estefanía, 46010 Valencia (ES); MARÍN SÁNCHEZ, Clotilde, 18071 Granada (ES); SÁNCHEZ MORENO, Manuel, 18071 Granada (ES); MARTÍN ESCOLANO, Rubén, 18071 Granada (ES); MARTÍN MONTES, Álvaro, 18071 Granada (ES)
(74) Representative: Elzaburu S.L.P.
(86) International application number: PCT/ES2021/070503
(87) International publication number: WO 2022/008784

(57) **Abstract**

The present invention provides the use of certain single acyclic polyamines of Formula (I), attached to heterocycles, for the treatment of leishmaniasis and trypanosomiasis. All the compounds studied show antiparasitic activity against *Leishmania* species, comparable or superior to that of the active ingredient of the drug commonly used to treat this disease, but with a macrophage toxicity similar to or, in most cases, lower than that of the commercial reference compound, and with higher selectivity indices against strains representative of the three clinical forms of the disease: cutaneous, mucocutaneous and visceral. The compounds show ability to inhibit parasite superoxide dismutase, generally at concentrations lower than those required to inhibit human superoxide dismutase. All this supports its usefulness for treating leishmaniasis and also trypanosomiasis.

## Description

### Technical field

The present invention relates to the use of compounds for the treatment of diseases caused by protozoan parasites of the family *Trypanosomatidae,* particularly protozoan parasites belonging to the genera *Leishmania* or *Trypanosoma.* More specifically, the invention relates to the use of heterocyclic aromatic compounds attached to simple acyclic polyamines for the treatment of such diseases.

### Description of the invention

Parasitism is the biological association wherein an organism ("the parasite") lives on (ectoparasite) or within (endoparasite) the body of another organism ("host" or "host") from which it obtains its nutrients. Thus, parasitism is a process whereby a species improves its ability to survive by using individuals of other species as host to meet its basic and vital needs. The result of such association is primarily beneficial to the parasite, which results in the improvement of its *reproductive fitness* and is generally detrimental to the reproductive fitness of the host.

Parasites can be classified into different groups based on their location in the host and the influence they exert thereon. Thus, parasites that live inside the host are called endoparasites and those that live outside are called ectoparasites. For their part, parasitoid parasites are characterised by their ability to cause the death of the host organism.

The family *Trypanosomatidae,* in particular, is a family of protozoa belonging to the order Kinetoplastida, an order that is characterised because its members present a single flagellum or kinetoplast. Although within the family a genus of free-living protozoa, such as *Proleptomonas* can be considered to be included, almost all of its members are exclusively parasites, mainly of insects. These parasites often have life cycles that include a secondary host, such as a plant or a vertebrate animal. Among the latter are genera that cause diseases in human beings, such as leishmaniasis (produced by species of the genus *Leishmania)* or trypanosomiasis (produced by species of the genus *Trypanosoma).* The most representative species of the latter genus are *Trypanosoma brucei* (which causes sleep sickness or African trypanosomiasis) and *Trypanosoma cruzi* (which causes Chagas disease or American trypanosomiasis, traditionally associated with Central and South America).

The survival of parasites of the family *Trypanosomatidae* has been shown to be closely linked to the ability of its superoxide dismutase enzyme (FeSOD) to evade damage caused by the host's toxic radicals [S. Ghosh. S. Goswami, S. Adhya, Biochem. J. 2003, 369, 447]. Thus, the parasite's FeSOD plays a relevant role as part of antioxidant defence in parasites that develop diseases such as Chagas disease *(Trypanosoma cruzi*) or Leishmaniasis *(Leishmania spp.) [*N. Le Trang, S. R. Meshnick, K. Kitchener, J. W. Eaton, J. Biol. Chem., 1983, 258, 125]*.*

Leishmaniasis or Leishmaniosis is an infectious and parasitic disease, caused by a hemoflagellate parasite of the family *Trypanosomatidae* belonging to the genus *Leishmania.* It is a tropical disease and is listed by the WHO as a "neglected tropical disease", which is usually present in developing countries and affects millions of people with low economic resources. [Leishmaniasis: https://www.who.int/news-room/fact- sheets/detail/leishmaniasis].

Furthermore, in recent years it has spread to countries previously free of the disease such as Paraguay or there have been new outbreaks in areas where it was believed to be controlled, such as Madrid, and in lagomorphs, a reservoir unknown to date. [Epidemiological Report: Córdoba, Argentina, no. 2142, http://www.reporteepidemiologico.com/wp-content/uploads2019/01/REC-2142.pdf,_N. Garcia, I. Moreno, J. Alvarez, M.L. de la Cruz, A. Navarro, M. Pérez-Sancho, T. Garcia-Seco, A. Rodríguez-Bertas, M.L. Conty, A. Toraño, A. Prieto, L. Domínguez, M. Domínguez, Evidence of Leishmania infantum infection in rabbits (Oryctolagus cuniculus) in a natural area in Madrid, Spain, Biomed Res Int. 2014 (2014) 318254. doi: 10.1155/2014/318254.]

It is an endemic disease in 98 countries and responsible for 20000 to 30000 deaths per year. One million new cases arise annually.

The disease is transmitted to humans and animals from the bite of female phlebotomine sand flies, in the Old World belonging to the genus *Phlebotomus* and in the New World to the genus *Lutzomya.*

The pathology varies depending on the species that infects the human host. There are 21 species of this genus, but only three main clinical forms. The most serious of all is visceral leishmaniasis (VL), which affects numerous internal organs, especially the spleen and pancreas, organs in which it causes enlargement and is the pathognomonic sign of this disease. In addition, this form also known as "kala-azar" causes severe anaemia and irregular fever. Its lethality rises to 95% when no or insufficient treatment is received. According to the October 2009 review by the *Center for Food Security* & *Public Health* at Iowa State University (http://www.cfsph.iastate.edu/Factsheets/en/leishmaniasis-en.pdf), human visceral leishmaniasis is caused primarily by the species *Leishmania donovani* and *Leishmania infantum* (which currently includes the subspecies formerly known as *L. chagasi*)*.* Sometimes, some species that generally cause cutaneous leishmaniasis, such as *L. tropica* and L. *amazonensis,* can also cause visceral leishmaniasis in human beings.

Another widely distributed form is cutaneous leishmaniasis (LC) that causes one or several skin lesions in areas near the bite, which when cured leave a perpetual scar. Sometimes, if the treatment is insufficient or does not exist, this type of clinical form can visceralise and give the clinical picture described above. Most *Leishmania species cause cutaneous* leishmaniasis in human beings. In the western hemisphere (including Europe and Africa), the main species that cause cutaneous leishmaniasis include *L. tropica, L. major* and *L. aethiopica,* while in the eastern hemisphere (including Latin America) *L. braziliensis, L. panamensis*/*L. guyanensis, L. shawi* and *L. peruviana* (which are considered members of the L. brazilensis complex), L. *mexicana, L. amazonensis, L. venezuelensis* (considered members of the *L. mexicana* complex), in addition to *L. lainsoni, L.* naiffi and *L. linderbergi stand out.* Furthermore, some strains of *L. infantum* can cause cutaneous leishmaniasis without affecting the internal organs.

The last known clinical form is found exclusively in Latin America, is known as mucocutaneous leishmaniasis (ML) and destroys connective tissue and facial mass, resulting in conspicuous deformations that sometimes cause social rejection in affected individuals. [Leishmaniasis, https://www.who.intlnews-room/fact-sheets/detail/leishmaniasis]. The main species that causes this clinical form is *L. braziliensis* and it can also be caused by *L. panamensis*/*L. guyanensis.*

The main reservoir of this disease is the dog, but any vertebrate mammal can be a disease reservoir, both jungle and peri-urban. Some wild animals, such as opossums, coatis and anteaters, among others, are asymptomatic carriers of the parasite, while some others, including members of the canid family such as the wolf or the fox, may suffer from the disease. Some domestic animals, including not only the dog, but also cats and equidae (horses, mules and donkeys), may show symptoms of the disease, although the difference between species that cause cutaneous and visceral syndromes is less clear than in human beings. For example, *L. infantum* can cause both visceral and cutaneous disease in dogs, and mainly cutaneous lesions in dogs and horses.

*Leishmania* specimens show two morphologies during their life cycle:
- Promastigote. Elongated shape with anterior flagellum. It is present in the intestine and salivary glands of the invertebrate that acts as a vector.
- Amastigote. Spherical shape and with a very short flagella, which does not protrude from the flagellar pocket, so that it is only appreciable under the electron microscope. It reproduces within macrophages and cells of the reticuloendothelial system of the host vertebrate. Infections occur in the skin (cutaneous), skin and mucous membranes (mucocutaneous), or in organs (visceral).

There is currently no effective vaccine or treatment that is effective; moreover, current treatments have many drawbacks, such as high toxicity, generation of resistance, great difficulty in complying with the administration schedule and high cost.

The most common treatments consist of the administration of antimony-based compounds belonging to the group of pentavalent antimonials. These drugs are meglumine antimoniate (Glucantime^{®}) and sodium stibogluconate (Pentostam^{®}). These drugs, in addition to being difficult to administer, are highly toxic to the liver and kidneys. Their widespread use and the difficulty of administration (intramuscular injection) have made it difficult to comply with the treatment, which has triggered resistance. All of this indicates the need for new and more effective treatments.

There is also a need for new more effective treatments in trypanosomiasis, diseases caused by parasites of the genus *Trypanosoma,* particularly in the case of Chagas disease (American trypanosomiasis). It is a tropical parasitic disease, usually chronic, caused by *Trypanosoma cruzi.* The natural reservoir is formed by armadillos, marsupials, rodents, bats and wild primates, in addition to certain domestic animals such as dogs, cats, even rats and guinea pigs. It is commonly transmitted to humans by hematophagous Triatonimae, such as *Triatoma infestans,* which transmits the parasite when it defecates on the bite that it itself has made itself for feeding. It can also be transmitted by transfusion of contaminated blood, by ingestion of food contaminated by the parasite or vertically from the infected mother to the foetus. The insect that transmits this disease can become infected if it bites a person who has the infection, and thus acquire the ability to continue to spread this parasite.

It is estimated that between 15 and 17 million people are infected with Chagas disease each year, of which about 50,000 die. The acute childhood stage is characterised by fever, lymphadenopathy, enlargement of the liver and spleen, and sometimes myocarditis or meningoencephalitis with a serious prognosis. In the chronic stage, which is reached by between 30% and 40% of all Chagas patients, there is usually severe diffuse cardiomyopathy, or pathological dilatation (megasyndromes) of the Oesophagus and colon, megaoesophagus and megacolon, respectively.

*Trypanosoma cruzi* has three different forms:
- Amastigote: spherical or oval, it is the reproductive form inside mammalian cells.
- Epimastigote: elongated and with the kinetoplast located anterior to the nucleus, it is the reproductive form in the digestive tract of invertebrates and in culture media.
- Tripomastigote: also elongated, but with the kinetoplast located posterior to the nucleus. It is found in the blood of mammals and is the infectious form thereof. This form does not divide.

The most widely used active compound currently used for the treatment of Chagas disease is benznidazole (BZN), a drug derived from nitroimidazole, which is also known for its activity against leishmaniasis and has been marketed in Latin America under different trade names such as Lafepe Benznidazol^{®} in Brazil or Radanil^{®} in Argentina. However, as in the case of the commercial drugs for the treatment of leishmaniasis mentioned above, these drugs are not very effective, mainly in the chronic phase (more than 30 days of treatment) of the disease and have a very high toxicity.

Thus, although leishmaniasis and Chagas disease are diseases caused by parasites that affect millions of people around the world, there are currently very limited and dubiously effective therapeutic remedies. Moreover, as explained, the ones used lead to serious problems, since their effectiveness is variable, as they are long and expensive treatments, and, in addition, they are associated with serious toxic effects.

As disclosed in document ES2414291 B2, the group of the present inventors has observed that scorpion-type polyamines (compounds with an azamacrocyclic nucleus from which arms with different aromatic functionalities hang) exhibit antiparasitic activity in relation to Chagas disease and Leishmaniasis, showing lower toxicity than meglumine antimoniate (Glucantime^{®}) and benznidazole. Findings of some of the present inventors are also disclosed in document ES2566228B1, relating to the use of pyrazole-derived esters, preferably proton-ionisable, and their salts for the treatment of diseases caused by parasites of the family *Trypanosomatidae,* providing evidence of their effectiveness against parasites of the genus *Leishmania* and against *Trypanosoma cruzi.* In both patent documents, the compounds' activity seems to be related to their ability to inhibit the superoxide dismutase (SOD) type enzymes of the parasites, barely inhibiting human CuZn-SOD. This is interesting because, as noted above, FeSOD plays a vital role as part of the antioxidant defence in parasites of the genus *Leishmania* and other parasites of the family Trypanosomatidae that also cause diseases in human beings, such as *Trypanosoma cruzi,* the cause of Chagas disease. The molecular dynamics studies carried out suggest that the active molecules block the superoxide access channel to the active centre containing the metal ion, an interaction that would also alter the network of hydrogen bridges established around the water molecule coordinated with Fe, altering basic parameters such as the normal redox potential of the Fe^{III}/Fe^{II} pair.

The incidence and relevance of the diseases mentioned above makes it necessary to use antiparasitic compounds capable of treating these diseases effectively at all stages of the process, including the chronic stage of Chagas disease, and which, in addition, are not toxic to patients, or, at least, which have a reduced toxicity with respect to the reference compounds currently used. In the specific case of leishmaniasis, the recent expansion of the disease and the absence of effective treatments make it a threat to public health, hence the already existing need for new treatments is even more pressing. Added to this is the fact that differences in the efficacy of the treatments have also been observed depending on factors such as the clinical form of leishmaniasis or the geographical region. Thus, although compounds such as those disclosed in the document ES2414291B2 seem to be an interesting alternative to the treatments currently applied for leishmaniais and Chagas disease, it is advisable to have different alternative treatments that can be used for the control of diseases caused by parasites of the family *Trypanosomatidae* such as those mentioned above.

The present invention provides a solution to such a problem.

### Summary of the invention

The present invention, in a first aspect, relates to a compound of Formula (I) 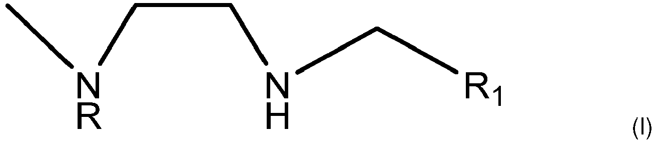 or a solvate or a pharmaceutically acceptable salt thereof,
where
R is selected from H or CH₃, and
R₁ is selected from the group of 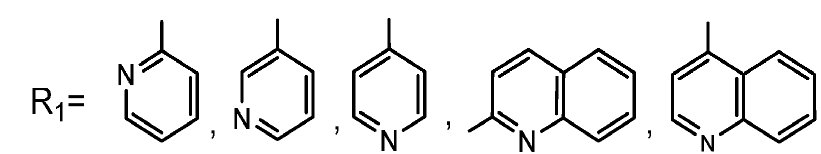
for its use in the treatment of a disease caused by a parasite of the family *Trypanosomatidae.* Preferably, the disease is selected from leishmaniasis and Chagas disease. It is particularly preferred that the disease is leishmaniasis. Leishmaniasis may be cutaneous, mucocutaneous, or visceral.

The compounds of Formula (I) thus defined, with the alternatives of R and R₁ just mentioned, are referred to throughout the remainder of the specification as the compounds of Formula (I) as defined above, the compounds of Formula (I) as defined herein, or, particularly in this "Summary of the Invention" section, the compounds of Formula (I) defined in the first aspect of the invention, since this is the first appearance of their definition.

As regards the subject to be treated, it may be in a mammal that can suffer from the disease, preferably by human beings. In the case of leishmaniasis, the subject can be selected from the group of dog, wolf, fox, cat, horse, mule, donkey, sheep, goat, cow and human being, preferably the latter, but also the dog.

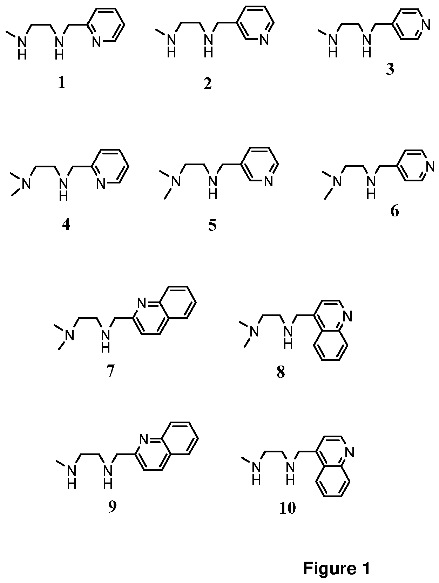

Preferably, the compound is selected from the group of compounds 1 to 10 shown in Fig. 1. More preferably, the compound is selected from the group of compounds 2 (1-{3-pyridyl)-2,5-diazahexane), 4 (1-(2-pyridyl)-5-methyl-2,5-diazahexane) and 5 (1-{3-pyridyl}-5-methyl-2,5-diazahexane), of said Fig. 1. Particular preference is given to compound 5.

In another aspect, the invention relates to a pharmaceutical or veterinary composition comprising at least one compound of Formula (I) indicated above, or a solvate or a pharmaceutically acceptable salt thereof, wherein
R is selected from H or CH₃, and
R₁ is selected from the group of 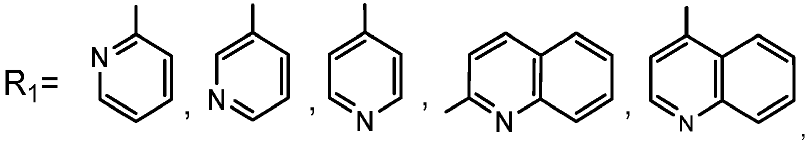
and, optionally, one or more pharmaceutically acceptable excipients and/or vehicles, for its use in treating a disease caused by a parasite of the family *Trypanosomatidae.* As in the case of the use of the compounds as such, the disease is selected from leishmaniasis and Chagas disease. It is particularly preferred that the disease is leishmaniasis. Leishmaniasis may be cutaneous, mucocutaneous, or visceral.

In a possible embodiment, the compound of Formula (I) is selected from the group of compounds of formulas 1 to 10 of Fig. 1.

In another possible embodiment, compatible with the previous, a pharmaceutical composition of the present invention comprises more than one compound of Formula (I) as defined in the first aspect of the invention, or more than one solvate or salt thereof.

Another aspect of the invention, closely related to the above, is a combined pharmaceutical or veterinary preparation comprising:
a) at least one compound of Formula (I) above, or a pharmaceutically acceptable salt or solvate thereof, wherein
   R is selected from H or CH₃, and
   R₁ is selected from the group of 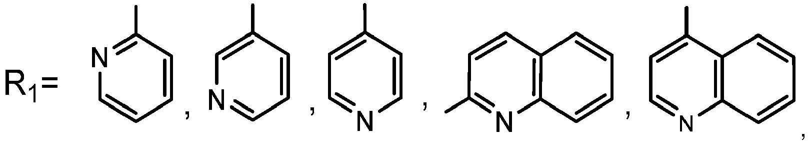
   or combinations thereof,
   and
b) at least one antiparasitic agent with activity against parasites of the family *Trypanosomatidae,* wherein the additional antiparasitic agent is different from the compounds defined in a),
for use in the treatment of a disease caused by a parasite of the family *Trypanosomatidae.*

Preferably, the antiparasitic agent is selected from the group of meglumine antimoniate, sodium stibogluconate, benznidazole and compounds for use as disclosed in documents ES2414291B2 or in document ES2566228B2 whose formula and/or name is expressly mentioned in any of said documents. Thus, a preferred embodiment is a combined pharmaceutical or veterinary preparation comprising
a) at least one compound of Formula (I) as defined above,
   and
b) at least one antiparasitic agent with activity against parasites of the family *Trypanosomatidae,* which is selected from the group of
   i. meglumine antimoniate,
   ii. sodium stibogluconate,
   iii. benznidazole,
   iv. a compound selected from the group of following compounds of formulas (II)sc, (III)sc, (IV)sc, (V)sc, (VI)sc, (VII)sc, (VIII)sc, (IX)sc, (X)sc, (Xl)sc, (XII)sc, (XIII)sc, (XIV)sc, (XV)sc, (XVI)sc, (XVII)sc, and (XVIII)sc: 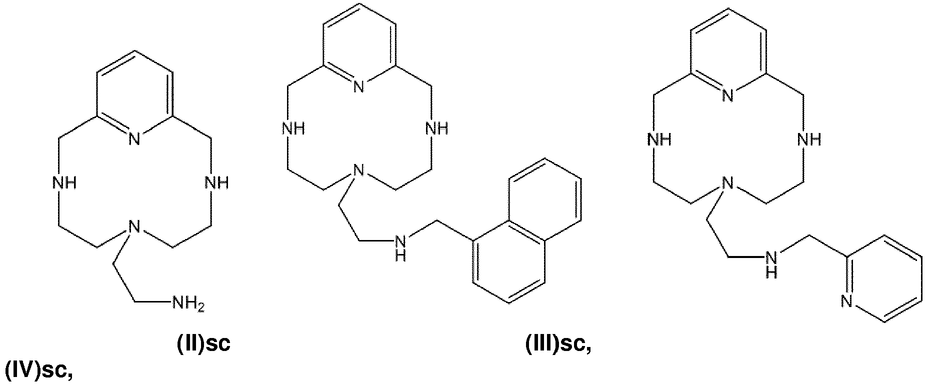 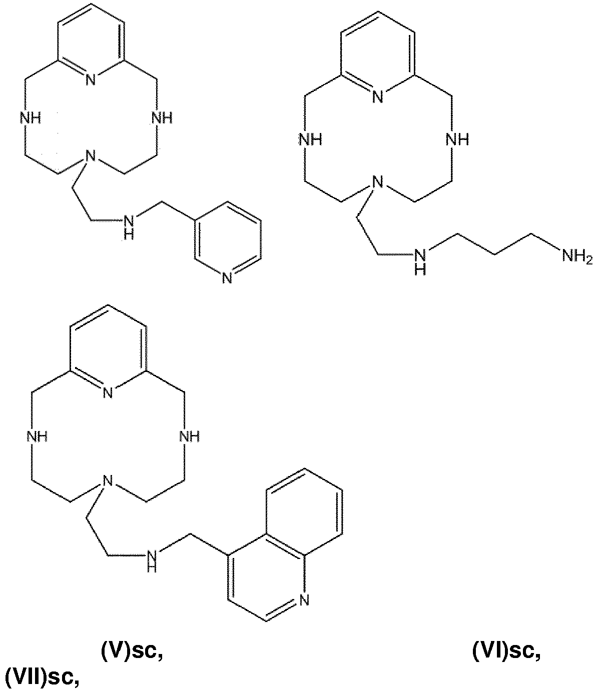 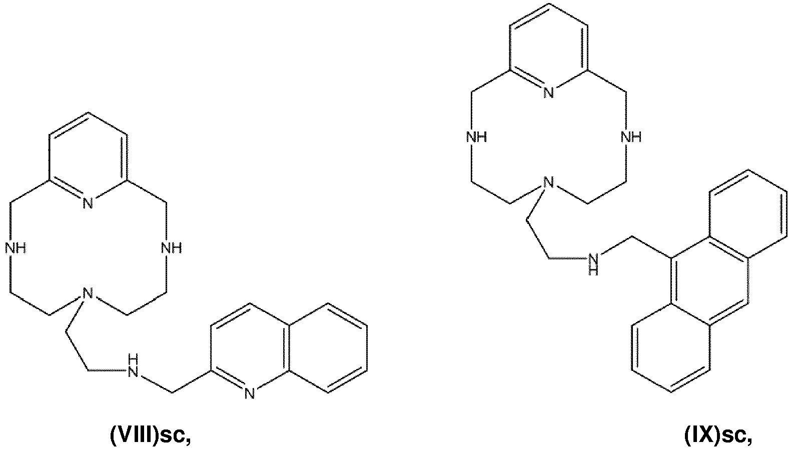 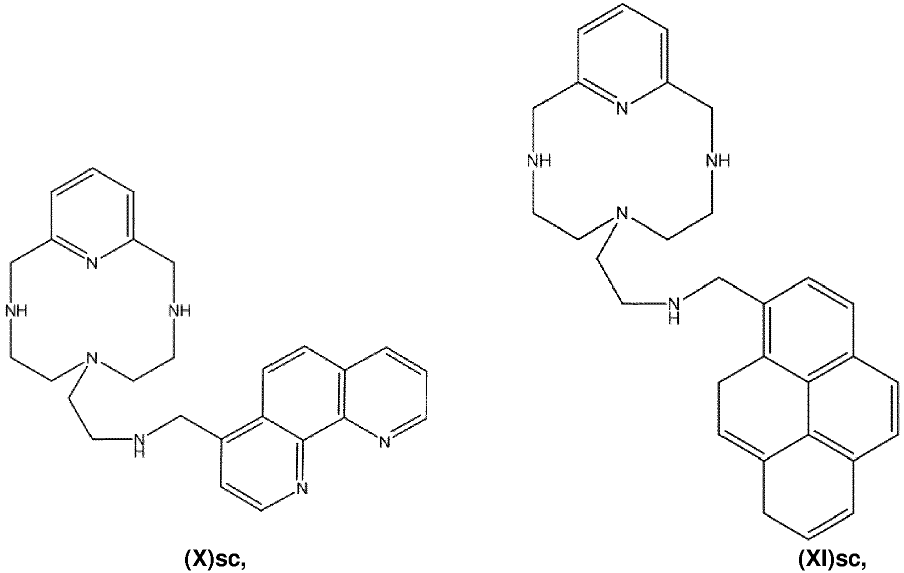 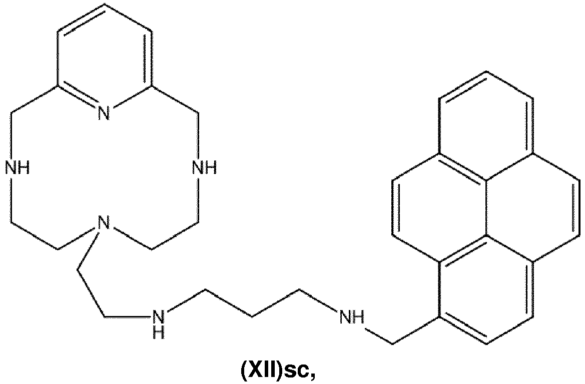 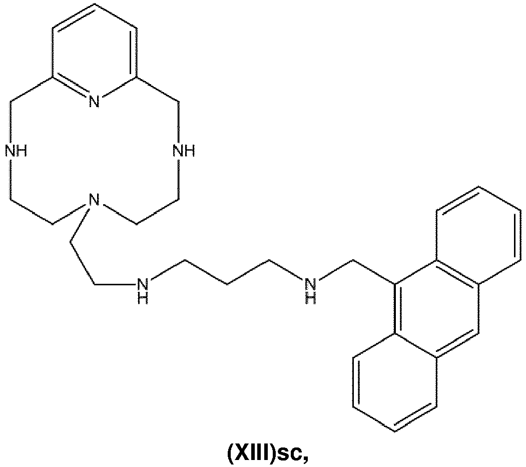 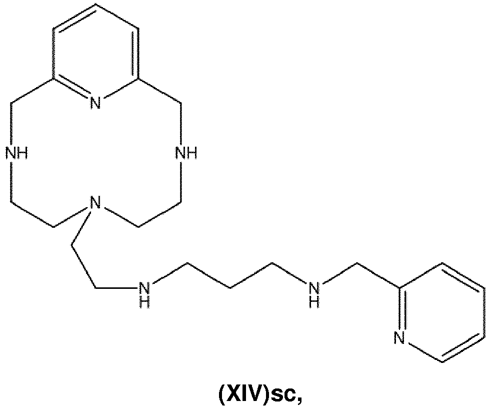 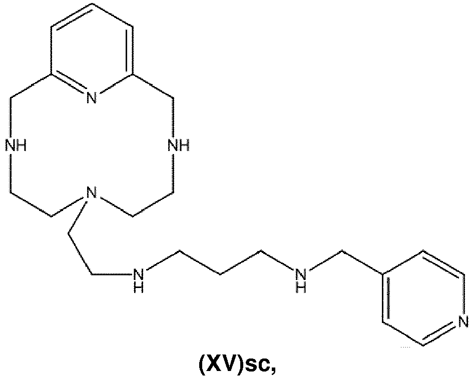 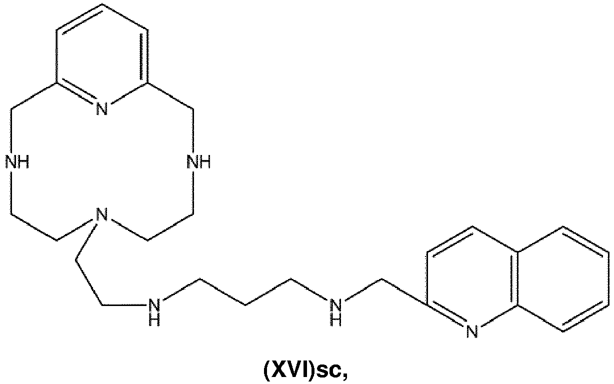 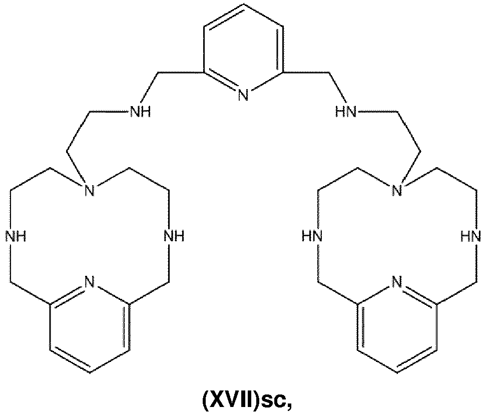 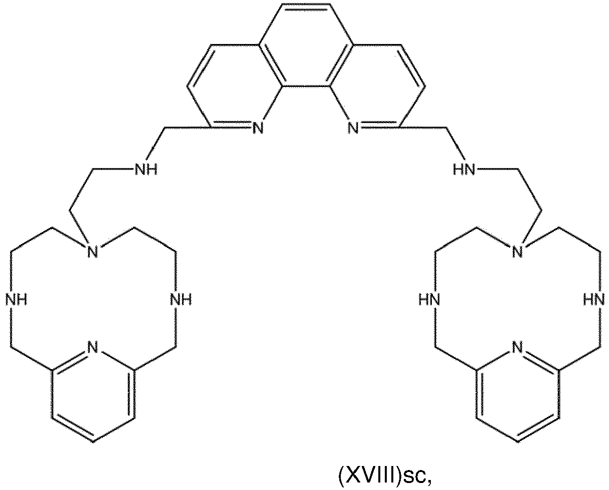 or
   v. a compound selected from the group of dimethyl 1*H*- pyrazole-3,5-dicarboxylate, sodium 3,5-bis(methoxycarbonyl) pyrazolate, diethyl 1A7-pyrazole-3,5-dicarboxylate, sodium 3,5-bis(ethoxycarbonyl) pyrazolate, dipropyl 1*H*-pyrazole-3,5-dicarboxylate, sodium 3,5-bis(propoxycarbonyl) pyrazolate, diisopropyl 1*H*-pyrazole-3,5-dicarboxylate and dibutyl 1*H*-pyrazole-3,5-dicarboxylate,
   or combinations thereof,
   for use in the treatment of a disease caused by a parasite of the family *Trypanosomatidae.*

As in the case of the compositions of the invention, in a possible embodiment, compatible with the previous, the compound of Formula (I) is selected from the group of compounds of formulas 1 to 10 of Fig. 1, and pharmaceutically acceptable salts or solvates thereof, or combinations thereof. The same preferences are given with respect to the compounds of Formula (I) as in the first aspect of the invention.

In another aspect, the invention relates to a kit-of-parts for preparing a combined pharmaceutical or veterinary preparation of the invention comprising at least one compound of Formula (I) above, or a pharmaceutically acceptable salt or solvate thereof, wherein
R is selected from H or CH₃, and
R₁ is selected from the group of 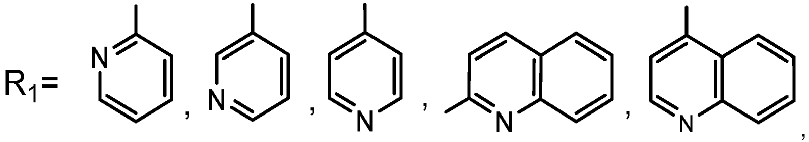
or combinations thereof,
and at least one antiparasitic agent with activity against a parasite of the family *Trypanosomatidae,* wherein the additional antiparasitic agent is different from the compounds whose use against said diseases is disclosed in the present invention, for use in the treatment of a disease caused by a parasite of the family *Trypanosomatidae.* Both with regard to the compound of Formula (I) and for the additional antiparasitic agent, the same preferences are given as in the case of a combined pharmaceutical preparation.

Aspects referring to a compound of Formula (I) as defined in the first aspect, or a solvate or a salt thereof, for its use according to the present invention, or a pharmaceutical or veterinary composition comprising at least one such compound for its use also according to the present invention, or a combined pharmaceutical or veterinary preparation of the invention for its use also according to the invention, or a kit-of-parts of the invention for its use according to the invention, can also be defined, or are related to, a method of treating a subject suffering from a disease caused by a protozoan parasite of the family *Trypanosomatidae,* comprising administering a therapeutically effective amount of a compound of Formula (I) as defined above, or a salt or solvate thereof, a pharmaceutical or veterinary composition of the invention, a pharmaceutical or veterinary preparation of the invention, or a kit-of-parts of the invention.

An aspect of the invention may also be considered the use of a compound of Formula (I) as defined in the first aspect, or a solvate or a salt thereof, of a pharmaceutical or veterinary composition of the invention, of a combined pharmaceutical or veterinary preparation of the invention, or of a kit-of-parts of the invention, for the preparation of a drug for the treatment of a disease caused by a parasite of the family *Trypanosomatidae.*

Whether it is considered a compound of Formula (I) as defined in the first aspect of the invention, or a solvate or a salt thereof, for its use in treating a disease caused by a parasite of the family *Trypanosomatidae,* or whether the aspect of the method of treating the disease or the aspect of preparing a drug for the treatment thereof is considered, the possibilities and preferences with respect to the disease to be treated, the subjects to be treated and the compounds (active ingredients) to be administered are the same. Thus, preferably, the disease is selected from leishmaniasis and Chagas disease, with particular preference for leishmaniasis, which can be selected, for example, from among leishmaniasis caused by a species of the genus *Leishmania* selected from among *L. tropica, L. major, L. aethiopica, L. braziliensis, L. panamensis*/*L. guyanensis, L. shawi, L. peruviana, L. mexicana, L. amazonensis, L. venezuelensis, L. lainsoni, L.* naiffi, L. linderbergi, *L. infantum and L. donovani* (with special preference for *L. braziliensis, L. infantum* and *L. donovani*) and/or from among the types of cutaneous, mucocutaneous or visceral leishmaniasis. The subject to be treated can be any mammal that may suffer from the disease, preferably by the human being in any of the possible diseases caused by a parasite of the family *Trypanosomatidae,* and possible subjects to treat for leishmaniasis being the mammals of the group of dog, wolf, fox, cat, horse, mule, donkey, sheep, goat, cow and human, preferably by the dog in the case of the aforementioned disease. And, in any of the aforementioned aspects, the alternatives with respect to the compound or compounds of Formula (I) (or its salts or solvates) to be included in the composition, the combined preparation or the kit-of-parts of the invention are the same, so that the preferences for the compounds of formulas 1 to 10 to form part of the compositions, combined preparations and kits of the invention, are joined by the particular preference for compound 5 or for one of the compounds of the group of 2, 4 and 5; as are also the possible alternatives and preferences with respect to the possible additional antiparasitic agents, which have already been mentioned in the preferences with respect to the combined preparations and the kits of parts of the invention.

The invention is now explained in greater detail, with the aid of the following detailed description, figures and Examples.

### Brief description of the figures

Figure 1. formulas of compounds 1 to 10 of the present invention.

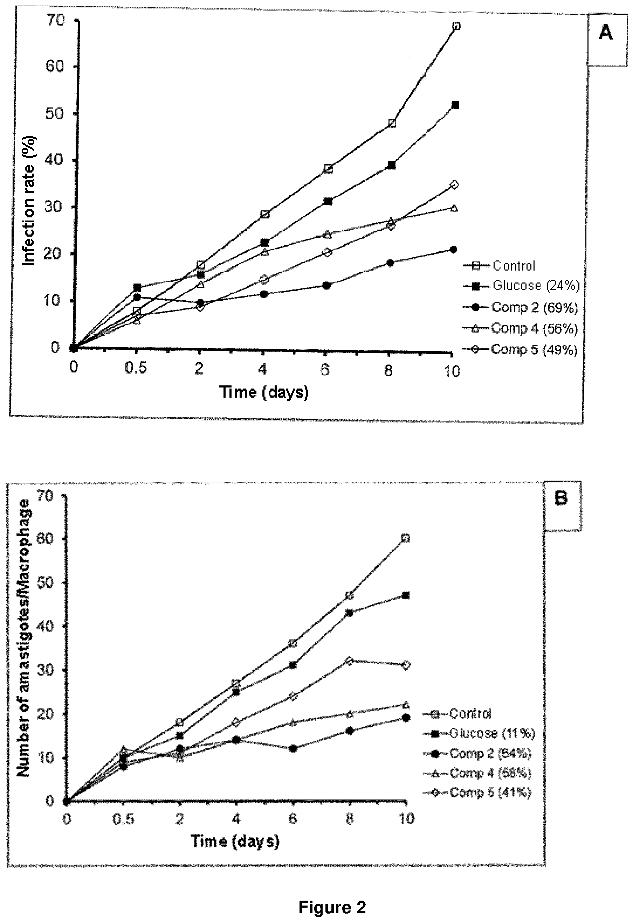

Figure 2. Evaluation of compounds 2 ("Comp 2"), 4 ("Comp 4") and 5 ("Comp 5") on the ability of infection and division of intracellular forms of *L. infantum* in culture of macrophages infected with *L. infantum* (A) Percentage of infection. (B) number of amastigotes per infected cell. Also included are data obtained with the reference drug, Glucantime^{®} ("Gluc" in the legends). The maximum percentage of reduction with respect to the control obtained after 10 days for the parameter indicated in the y-axis in each of the graphs is indicated next to the abbreviation of each compound.

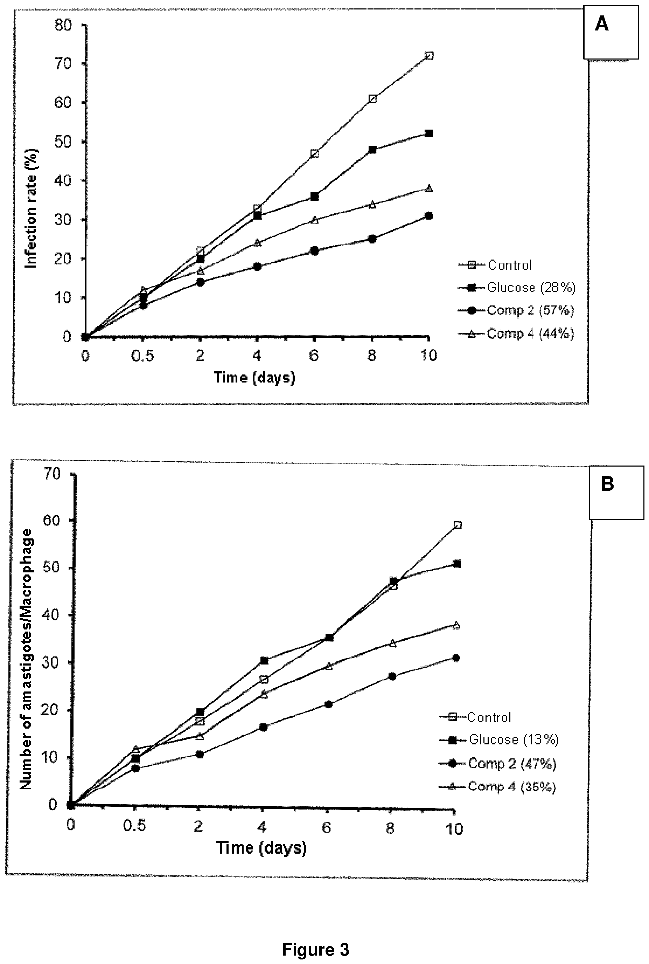

Figure 3. Evaluation of compounds 2 ("Comp 2") and 4 ("Comp 4") on the ability of infection and division of intracellular forms of *L. braziliensis* in culture of macrophages infected with L. *braziliensis* (A) Percentage of infection. (B) Number of amastigotes per infected cell. Also included are data obtained with the reference drug, Glucantime^{®} ("Gluc" in the legends). The maximum percentage of reduction with respect to the control obtained after 10 days for the parameter indicated in the y-axis in each of the graphs is indicated next to the abbreviation of each compound.

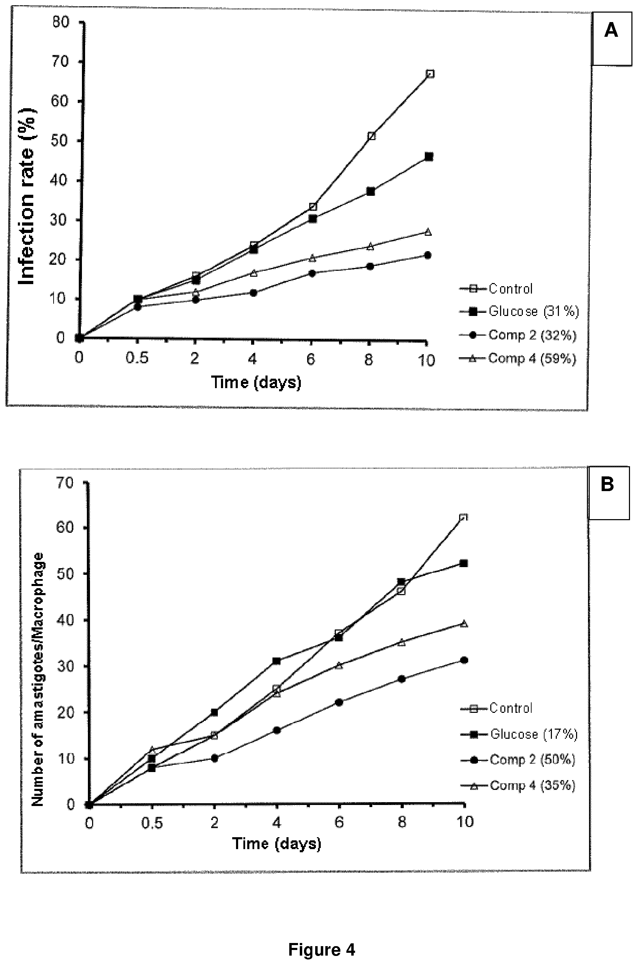

Figure 4. Evaluation of compounds 2 ("Comp 2") and 4 ("Comp 4") on the ability of infection and division of intracellular forms of *L. donovani* in culture of macrophages infected with L. *donovani* (A) % infection. (B) Number of amastigotes per infected cell. Also included are data obtained with the reference drug, Glucantime^{®} ("Gluc" in the legends). The maximum percentage of reduction with respect to the control obtained after 10 days for the parameter indicated in the y-axis in each of the graphs is indicated next to the abbreviation of each compound.

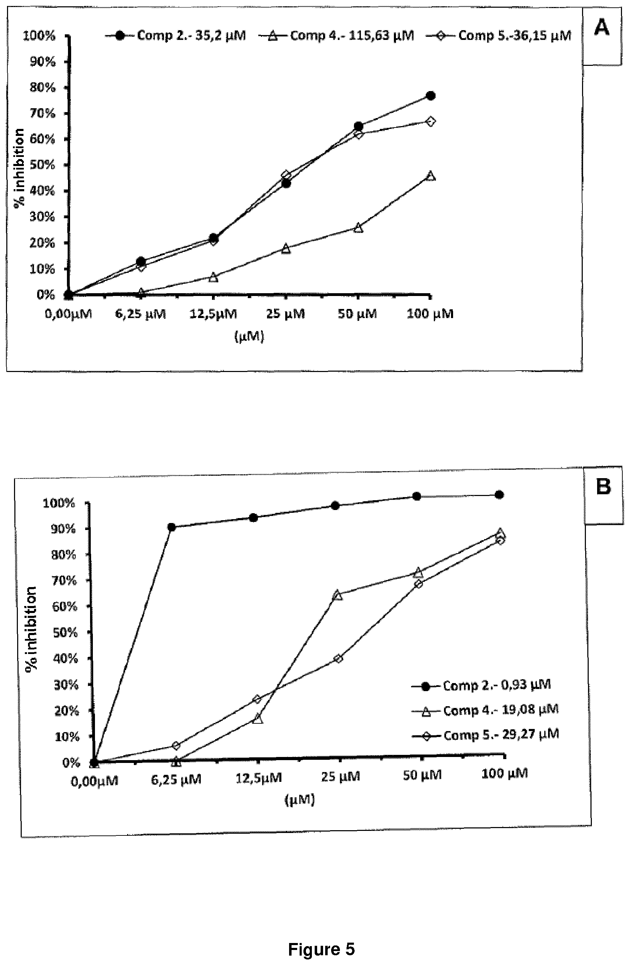

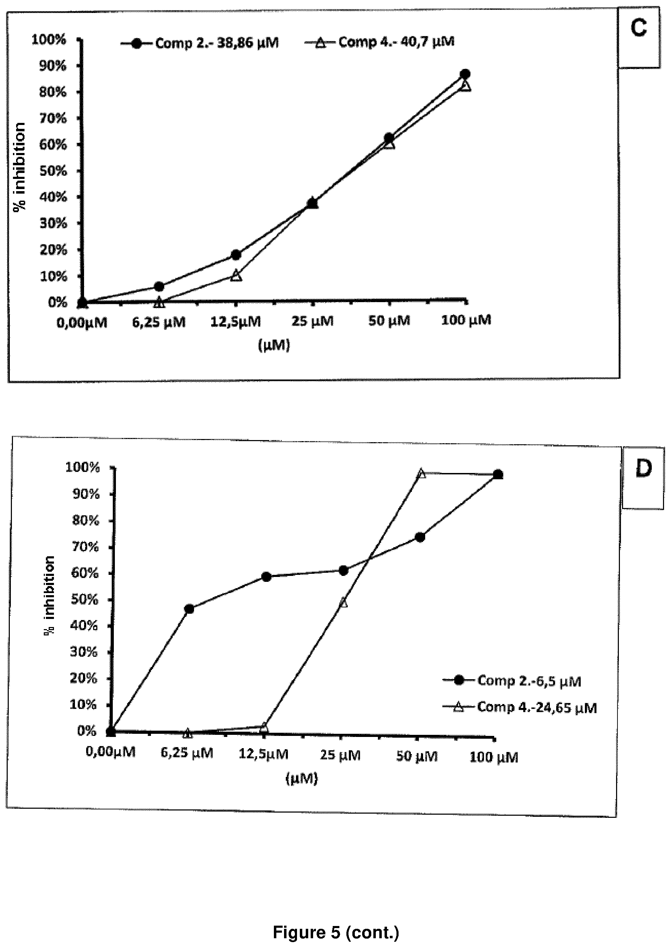

Figure 5. (A) In vitro inhibition (%) of CuZnSOD of human erythrocytes with compounds 2 ("Comp 2"), 4 ("Comp 4") and 5 ("Comp 5"). (B) In vitro inhibition (%) of FeSOD of promastigote forms of *L. infantum* with compounds 2 ("Comp 2"), 4 ("Comp 4") and 5 ("Comp 5"). (C) In vitro inhibition (%) of FeSOD of the promastigote forms of *L. braziliensis* with compounds 2 ("Comp 2") and 4 ("Comp 4"). (D) In vitro inhibition (%) of FeSOD of promastigote forms of *L. donovani* with compounds 2("Comp 2") and 4 ("Comp 4").

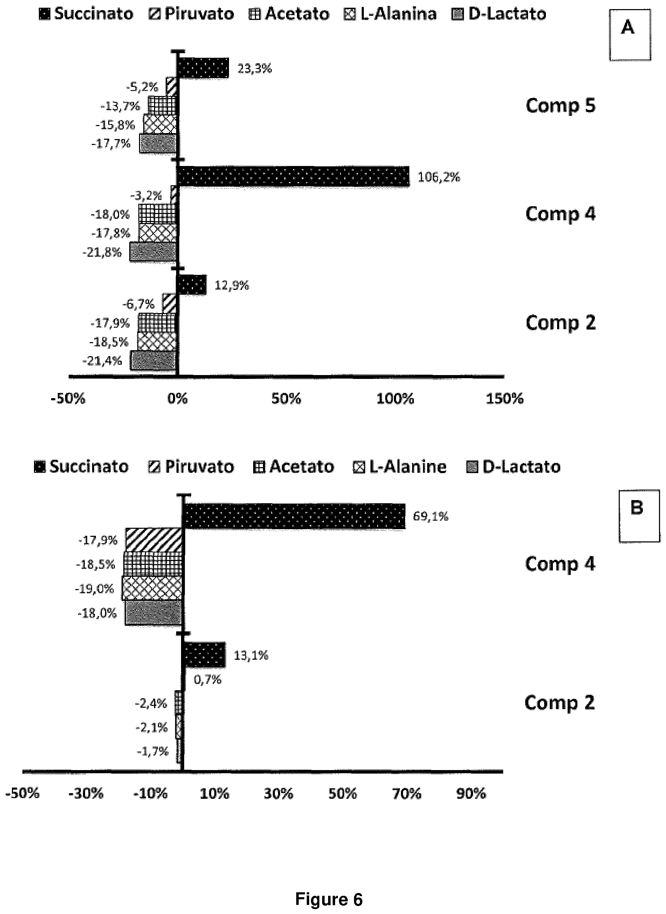

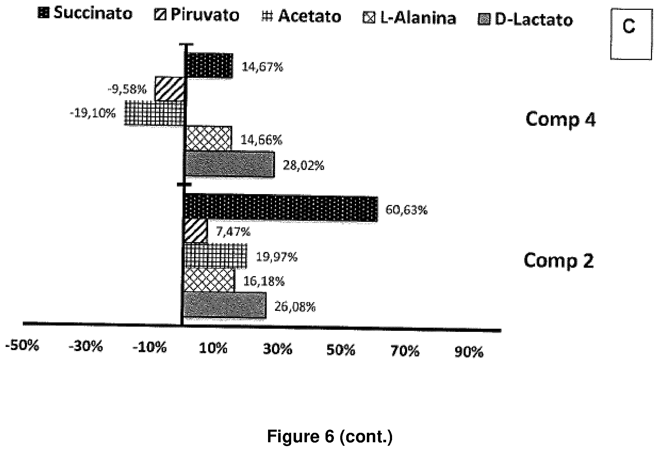

Figure 6. (A) Impaired production and excretion of *L. infantum* metabolites caused by compounds 2, ("Comp 2"), 4 ("Comp 4") and 5 ("Comp 5"). (B) Altered production and excretion of *L. braziliensis* metabolites caused by compounds 2 ("Comp 2" and 4 ("Comp 4"). (C) Altered production and excretion of *L. donovani* metabolites caused by compounds 2 ("Comp 2") and 4 ("Comp 4").

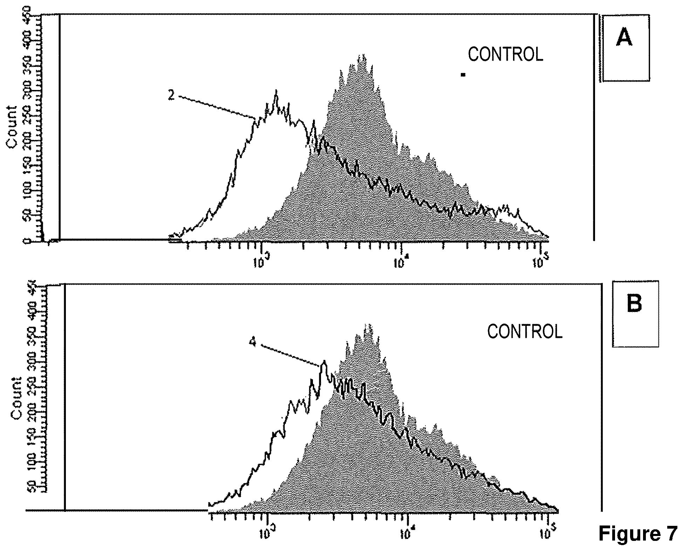

Figure 7. Effect of Compounds 2 (A) and 4 (B) on mitochondrial membrane potential of *L. donovani.*

### Detailed description of the invention

As previously indicated, the present invention relates to certain compounds of Formula (I), as well as to their possible pharmaceutically acceptable salts or solvates, for use in the treatment of diseases caused by parasites of the family *Trypanosomatidae,* such as Chagas disease and leishmaniasis. Such compounds of Formula (I) exhibit aromatic heterocycles attached to single acyclic polyamines.

Despite the difference in structure with, for example, the scorpion-like compounds disclosed in ES2414291B2or the pyrazole-derived esters, preferably proton-ionisable, described in ES2566228B1, the assays described below in the Examples of the present application show that these compounds, and in particular compounds 1 to 10 whose synthesis is described in Example 1, have considerable antiparasitic activities against parasites of the genus *Leishmania.* The antiparasitic activity seems to be related to its ability to inhibit the superoxide dismutase (SOD) type enzymes of parasites, barely inhibiting human CuZn-SOD, as also previously disclosed for the compounds of documents ES241491B2 and ES2566228B1, where antiparasitic activity was shown not only against protozoa causing leishmaniasis, but also against another parasite of the family *Trypanosomatidae, Trypanosoma cruzi,* which causes Chagas disease. This coincidence in the mode of action, despite the structural difference of the compounds for antiparasitic use of the present application with those of the two mentioned documents, not only makes the findings of the present invention surprising and unexpected, but also makes it plausible to extend the use of the compounds of Formula (I) as defined in the present application, (and that of the compositions and combined preparations comprising them or the kits of parts for preparing the latter) to the treatment of diseases produced by other parasites belonging to the family *Trypanosomatidae,* which also present superoxide dismutase (SOD) type enzymes, specifically FeSOD enzymes with Fe in the active centre, and wherein said enzyme seems to play a fundamental role as part of the antioxidant defence, so that the compounds capable of inhibiting said enzyme should have positive effects in the treatment of the diseases produced by said parasites, also with low toxicity to the host. That is why the scope of the present invention comprises the compounds of Formula (I), as defined in the present application, for its use in the treatment of diseases produced by parasites belonging to the family *Trypanosomatidae,* thus including both leishmaniasis and trypanosomiasis such as Chagas disease.

In addition, as can be seen in Example 2, the toxicity data in macrophages are similar or, in most cases (compounds 3, 4, 5, 6, 7 and 9), much lower than those obtained with the reference compound, meglumine antimoniate (the active ingredient of the drug marketed as Glucantime^{®}), which is one of the most commonly administered treatments against leishmaniasis, but which is toxic to the liver and kidneys. The compounds for use according to the present invention, and more specifically, all compounds 1 to 10 with which assays have been performed, also have values of the selectivity index against macrophages several times higher than those of the reference compound, for all the strains assayed (strains of *L. infantum, L. braziliensis* and *L. donovani*) and for both forms thereof: amastigote and promastigote. Furthermore, these compounds are prepared in such a way that the compounds, or salts or hydrates thereof, can be obtained on a large scale in a working day. For all these reasons, the compounds for use according to the present invention, and particularly compounds 1 to 10 and their solvates (preferably hydrates) or pharmaceutically acceptable salts, represent an interesting alternative to the commonly used treatments and even in addition to the compounds disclosed in documents ES2414291B2 and ES2566228B1.

As used in the present application, the term "solvate" also includes the term "hydrate", i.e. a compound formed by the addition of water or its elements to a receptor molecule. Hydrates are the preferred solvates for the purposes of the present invention, in any of its embodiments. As can be seen in Example 1, for example, the analysis data performed on compounds 1, 2, 4, 9 and 10 correspond to hydrates of such compounds. The term "salt", for its part, as used in this application, also includes acid addition salts, such as hydrochloride salts, which are the form wherein, for example, compounds 1 and 4 are obtained following the methodology described in Example 1 of this application.

As can be seen in Example 2 of the present application, due to its toxicity in macrophages, lower than that of the reference compound, a preferred embodiment of the invention can be considered that wherein the compound is selected from the group of compounds 3, 4, 5, 6, 7 and 9 of Fig. 1 and their pharmaceutically acceptable salts and solvates.

On the other hand, taking into account the results of the assays performed with respect to the selectivity indices, both for amastigote and promastigote forms, with strains of *L. infantum,* L. braziliensis and *L. donovani,* those that refer to the use of compounds 2, 4 (with good results for the three strains) and also 5 (for their good selectivity index results with respect to L. *infantum)* of the compounds represented in Fig. 1. As can be seen in Example 3, any of these three compounds decreases the rate of infection and the number of amastigotes per macrophage to a greater extent than the reference drug, the active ingredient of Glucantime^{®}. In assays with the *L. infantum* strain MCAN/ES/2001/UCM-10, isolated from dogs and known to cause the cutaneous form of leishmaniasis in humans, the three compounds showed better results than the reference drug. Compounds 2 and 4 are also effective by decreasing the infection rate and the number of amastigotes per macrophage of the other two strains, L. *braziliensis* strain MHOM/BR/1975/M2904 and *L. donovani* strain LCR-L 133, which are representative, respectively, of the mucocutaneous and visceral form of leishmaniasis in human beings.

Thus, although in principle any of the compounds of the present invention can be considered an alternative to the compounds used to date for the treatment of leishmaniasis caused by any species of the genus *Leishmania* (for example, selected from the group of *L. tropica, L. major, L. aethiopica, L. braziliensis, L. panamensis*/*L. guyanensis, L. shawi, L. peruviana* , *L. mexicana, L. amazonensis, L. venezuelensis, L.* lainsoni, L. naiffi, *L. linderbergi, L. infantum, L. donovani*)*,* preferred embodiments of the invention are those wherein compound 2, 4 or 5 is used for the treatment of leishmaniasis caused by L. *infantum,* and compound 2 or 4 for the treatment of leishmaniasis caused by *L. braziliensis,* or for leishmaniasis caused by *L. donovani.*

The assays shown below in Examples of the present application show that the leishmaniasis to be treated may be visceral leishmaniasis, cutaneous leishmaniasis, or mucocutaneous leishmaniasis. It may be caused by any hemoflagellate protozoa belonging to the genus *Leishmania,* such as those of the species *L. infantum, L. braziliensis* and *L. donovani.* The treatment may be for application in mammals that may suffer the disease, such as human beings, cats *(Felis silvestris catus,* also known as *Felis silvestris domesticus* or *Felis catus),* equidae (horses, donkeys and mules, i.e. individuals of the species *Equus ferus caballus and Equus africanus asinus* and the individuals resulting from the crossing of both species), sheep *(Ovis orientalis aries* or, simply, *Ovis aries),* goats *(Capra aegagrus hircus* or, simply, *Capra* hicus), cows and bulls *(Bos taurus)* and mammals of the canid family, preferably the dog *(Canis familiaris)* and the wolf *(Canis lupus),* but also foxes (*Vulpes vulpes*) and others such as the coyote *(Canis latrans)* or the jackal *(Canis aureus).*

Due to the more favourable results obtained with strains characteristic of this type of clinical manifestation of leishmaniasis in human beings, a compound selected from 1-(3-pyridyl) -2,5-diazahexane (compound 2), 1-(2-pyridyl) -5-methyl-2,5-diazahexane (compound 4) and 1-(3-pyridyl) -5-methyl-2,5-diazahexane (compound 5) is preferred for its use in the treatment of cutaneous leishmaniasis; a compound selected from 1-(3-pyridyl) -2,5-diazahexane (compound 2) and 1-(2-pyridyl) -5-methyl-2,5-diazahexane (compound 4) for its use in the treatment of mucocutaneous leishmaniasis, and a compound selected from 1-(3-pyridyl) -2,5-diazahexane (compound 2) and 1-(2-pyridyl) -5-methyl-2,5-diazahexane (compound 4) for its use in the treatment of visceral leishmaniasis, all in human beings.

If the mammal is a dog, which normally develops leishmaniasis caused by *L. infantum,* it is preferred that the compound is selected from compounds 2, 4 and 5, which have shown good results with the strain of this species in the Examples shown below.

The usefulness of compounds 2, 4 and 5 in particular is supported by additional assays, wherein the inhibition of the FeSOD enzyme of the promastigote forms of the three *Leishmania* strains used in the present assays has been studied, against the concentration necessary to inhibit human blood superoxide dismutase, the CuZnSOD of human erythrocytes. These assays are particularly interesting because the FeSOD enzyme is exclusive to the kinetoplastid parasites whereto *Leishmania* belongs, being absent from the vertebrate host, which usually has an analogous form of this enzyme but complexed with a copper or zinc atom (Cu-ZnSOD). For this reason, FeSOD is a therapeutic target for the design of new drugs, since the capture of the metal ion by complexing mechanisms can inactivate the enzyme and compromise the survival of the parasite before the appearance of free radicals. As can be seen in Example 4, compounds 2, 4 and 5 showed ability to inhibit FeSOD of the promastigote forms of the three *Leishmania* strains used in the assays of this application; furthermore, in almost all cases, the assayed compounds are capable of inhibiting the parasite FeSOD enzyme with an IC₅₀ value lower than that required for the human erythrocyte enzyme. It is worth highlighting, on the one hand, the low IC₅₀ value of compound 2 for the *L. infantum* strain and the *L. donovani* strain. Also noteworthy is the high IC₅₀ value of compound 4 with respect to the CuZnSOD of human erythrocytes, more than twice the IC₅₀ of the same compound observed in the assays with L. *braziliensis* and almost an order of magnitude higher than the values obtained in the assays with *L. infantum* and *L. donovani.* These differences show that the parasite is affected before there is a significant inhibition of the human host enzyme, which allows considering its use to be administered to patients affected by the parasite and, as discussed above, to patients affected by diseases produced by other parasites of the *Trypanosomatidae* family, such as Chagas disease.

The three compounds selected for complementary assays (2, 4 and 5) also give rise to alterations in glucose metabolism of the three strains studied, with the increase in succinate production versus control being significant.

Therefore, the assays performed support the usefulness of the compounds of the invention for the treatment of leishmaniasis and other diseases produced by parasites of the family *Trypanosomatidae.* The simplicity of their synthesis method, as proposed in the following application, and the ease of its scaling for industrial production, as already mentioned at the beginning, is also an advantage that makes them an interesting alternative to current treatments.

The compounds, or pharmaceutically acceptable salts or solvates thereof, will be administered in the form of a pharmaceutical or veterinary composition, with pharmaceutically or veterinary acceptable excipients. The oral route is one of the possible routes of administration, for example in the form of tablets, lozenges or capsules, but also in the form of syrups or other liquid forms designed to be ingested. Topical or intradermal injection is also considered.

The present invention covers pharmaceutical or veterinary compositions comprising at least one compound of Formula (I) as defined herein, preferably in a therapeutically effective amount, for its use in treating a disease caused by a parasite of the family *Trypanosomatidae.* Optionally, such compositions may also contain pharmaceutically acceptable excipients and/or vehicles. In the present invention, a therapeutically effective amount is understood to be one that reverses the treated disease or improves its symptoms.

Also contemplated within the scope of the present invention are the combined pharmaceutical or veterinary preparations comprising at least one compound of Formula (I) as defined herein, or a salt or solvate thereof, and additionally at least one antiparasitic agent having activity against a parasite of the family *Trypanosomatidae,* wherein the additional antiparasitic agent is different from the compounds the use of which against said diseases is disclosed in the present invention. The additional antiparasitic agents can be selected from the group of those already used for the treatment of leishmaniasis and/or trypanosomiasis (such as meglumine antimoniate, sodium stibogluconate, benznidazole) or also others, such as the compounds for which antiparasitic activity against parasites of the *Trypanosomatidae* family is disclosed in patent documents ES2414291B2 or in document ES2566228B2. The combined pharmaceutical or veterinary composition may comprise more than one compound of Formula (I) as defined herein, or salts or solvates thereof, and more than one additional antiparasitic compound. In the latter case, it can be considered a case of "combined pharmaceutical or veterinary preparation."

As used in the present application, the term "combined pharmaceutical or veterinary preparation" includes compositions, formulations, preparations, or pharmaceutical or veterinary forms and is compatible with the "juxtaposition" of the components thereof, i.e. it is not necessary for such components to be present forming a "true" composition in its strictest sense (e.g. dissolved or in solution in the same liquid vehicle or integrated into the same solid carrier or vehicle or forming part of the same particle or solid form), but may be physically separate and not integrated into the same solid or liquid form or presentation. Thus, the term "juxtaposition" does not necessarily imply a true combination of the components, although it is compatible therewith, so that the combined preparations of the present invention include in their definition possible embodiments of the pharmaceutical or veterinary compositions of the present invention defined above. This broad definition of the term "combined pharmaceutical or veterinary preparation", contemplates that the components thereof are available for its combined or separate, simultaneous or sequential application, as deemed most convenient. Thus, a combined pharmaceutical or veterinary preparation comprising a compound of Formula (I) as defined herein, or a solvate or a salt thereof, and an anti-parasitic agent having activity against a parasite of the family Trypanosomatidae, for simultaneous, separate or sequential use in the treatment of a disease caused by a parasite of the family *Trypanosomatidae* may be considered to be within the scope of the invention, it being preferred that the disease be selected from Chagas disease and leishmaniasis, with particular preference for the latter.

As can be appreciated, the concept of a combined pharmaceutical or veterinary composition is related to that of a "kit-of-parts"may comprise the components necessary to obtain a combined pharmaceutical or veterinary preparation. As used herein, a kit-of-parts relates to a set of components suitable for obtaining a composition which, for the purposes of the present invention, will be a pharmaceutical or veterinary composition of the invention or, more preferably, a combined pharmaceutical or veterinary preparation of the present invention. Kit components may or may not be packaged together, although each component may be in its own container and packaging appropriate for commercial distribution and/or appropriate to facilitate subsequent use or storage. Thus, a kit-of-parts for its use according to the present invention will comprise at least one compound of Formula (I) as defined herein and preferably also at least one anti-parasitic agent having activity against parasites of the family *Trypanosomatidae,* analogously to that of the combined pharmaceutical or veterinary preparations. Preferably, a kit-of-parts for use according to the present invention is for preparing a combined pharmaceutical or veterinary preparation of the present invention.

Further, as previously mentioned, one aspect of the present invention is the use of a compound of Formula (I) as defined in the present application, or a solvate or a salt thereof, a pharmaceutical or veterinary composition of the invention, a combined pharmaceutical or veterinary preparation of the invention and/or a kit-of-parts of the invention for the manufacture of a drug for the treatment of a disease caused by a parasite of the family *Trypanosomatidae.* In a particular embodiment, the drug is suitable for the treatment of Chagas disease and/or leishmaniasis or diseases caused by parasites of the genus *Leishmania,* preferably parasites of the species *L. infantum, L. donovani* or *L. braziliensis,* and more preferably for the treatment of cutaneous, mucocutaneous, visceral and/or canine leishmaniasis.

Closely linked to this is also the aspect referring to a method of treating a subject suffering from a disease caused by a protozoan parasite of the family *Trypanosomatidae* comprising administering a therapeutically effective amount of at least one compound of Formula (I) as defined in the present application, or a solvate or a salt thereof, or a pharmaceutical or veterinary composition of the invention, a combined pharmaceutical or veterinary preparation of the invention, or a kit-of-parts of the invention. In this aspect, the use of a combined pharmaceutical or veterinary preparation of the invention facilitates the simultaneous, separate or sequential administration of the compound of Formula (I) as defined in the present application, or of a solvate or a salt thereof, and the additional antiparasitic agent which is also a component of said preparation, leaving a wider margin for different treatment guidelines as may be considered appropriate. In any case, the subject will receive a therapeutically effective amount of at least one compound of Formula (I) as defined herein, or of a solvate or salt thereof.

The effects of the treatment method of the present invention include, but are not limited to, the effects of disease elimination, the increase in disease progression time, and the survival rate. The effects of treatment include, in the longer term, the control of the disease.

The invention will now be explained in greater detail by means of the following Examples and Figures.

### Examples

### Example 1: Synthesis of compounds.

All compounds were synthesised by modifying the procedure described for the synthesis of compounds 1 and 4 in the following references: J. A. R. Hartman, R. W. Vachet, J. H. Callahan, Gas, solution, and solid state coordination environments for the nickel(II) complexes of a series of aminopyridine ligands of varying coordination number, Inorganica Chimica Acta 297 (2000) 79-87); and A. Raja, V- Rajendira, P. U. Maheswari, R. Balamurugan, C. A. Kilner, M. A. Halcrow, M. Palaniandavar, Copper(II) complexes of tridentate pyridylmethylethylenediamines: Role of ligand steric hindrance on DNA binding and cleavage, J. Inorg. Biochem. 99 (2005) 1717-1732, F. GroB, A. Müller-Hartmann, Heinrich Vahrenkamp, Zinc Complexes of Condensed Phosphates, 3[*1 Diphosphate-Zinc Complexes with Tridentate Coligands, Eur. J. Inorg. Chem. 2000, 236322370; H-D. Bian, J.-Y. Xu, W. Gu, S-P. Van, D.-Z. Liao, Z.-H. Jiang, P. Cheng, Synthesis, structure and properties of terephthalate-bridged copper (11) polymeric complex with zigzag chain, Inorg. Chem. Commun. 6 (2003) 573-576).

The synthesis of the compounds consisted in the reaction of the appropriate carboxaldehyde with the corresponding diamine, in dry EtOH as a solvent (instead of methanol). The resulting mixture was stirred for 2 hours at room temperature, and then, an excess of sodium borohydride was added in portions for the reduction of the obtained imine (replacing the hydrogenation with Pd described in the literature). After 2 hours, the solvent was evaporated to dryness. The residue was treated with water and repeatedly extracted with CH₂Cl₂ (3 × 40 ml). The organic phase was collected and dried with anhydrous MgSO₄ and the solvent was evaporated to dryness to give an oil. The oil was then recovered with a minimal amount of EtOH and precipitated with HCl in dioxane to obtain the hydrochloride salt, form in which the compounds were preserved. The latter represents another variation on the methodology collected in the literature, where free amines are obtained, generally more difficult to preserve.

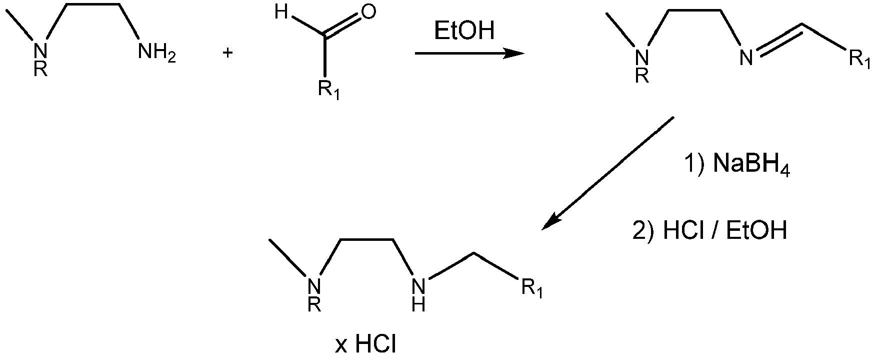

R= H, CH₃

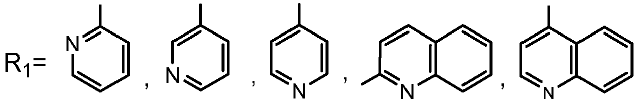

### Diagram 1. General synthesis procedure.

Compounds 1 to 10 were synthesised, whose formula is indicated below, were synthesised by means of the process indicated and which has also been reproduced in Fig. 1. The results of the analysis of each one are also indicated below.

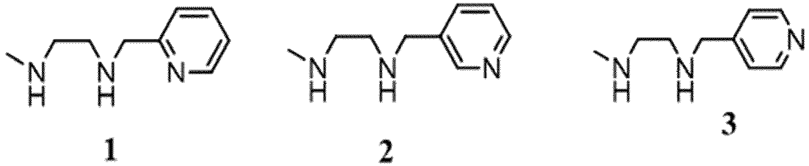

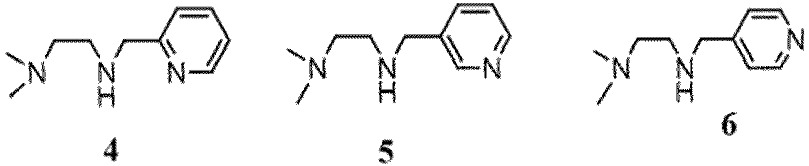

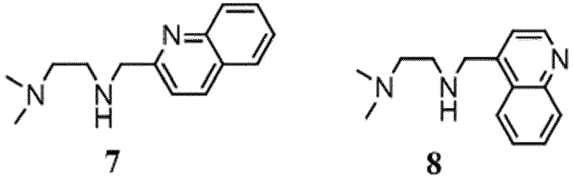

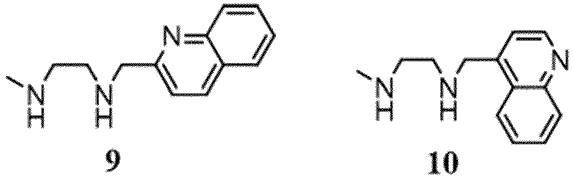

***1-{2-pyridyl}-2,5-diazahexane.* (1).** C₉H₁₈N₃Cl₃ · 2H₂O (Molecular weight = 312.89 g/mol). Yield: 63.7 %. ¹H NMR (300 Hz, D₂O): δ_{H} (ppm): 2.78 (s, 3H), 3.48 - 3.51 (m, 2H), 3.59-3.64 (m, 2H), 4.67 (s, 2H), 7.92 - 7.96 (m, 1H), 8.015 (d, *J* = 8.1 Hz, 1H), 8.46 (t, *J =* 8.0Hz, 1H), 8.795 (d, *J=* 5.6Hz, 1H). ¹³C NMR (75 MHz, D₂O) δ: 146.99, 145.68, 143.96, 126.55, 126.36, 49.22, 44.49, 43.25, 33.21. Melting point: 165.6 ºC. Elemental analysis: Calculated: C 34.79%; H 7.13%; N 13.52% Experimental: C 35.05%; H 6.74%; N 14.72%.

***1-{3-pyridyl)-2,5-diazahexane.* (2).** C₉H₁₇N₃Cl₂ · 2H₂O (Molecular weight = 274.18 g/mol). Yield: 62.36 %. ¹H NMR (300 Hz, D₂O): δ_{H} (ppm): 2.81 (s, 3H), 3.43 (s, 4H), 4.34 (s, 2H), 7.68 (dd, *J* = 8.1 Hz, *J* =5.2Hz, 1H), 8.14 (d, *J=* 8.2Hz, 1H), 8.65 - 8.69 (m, 2H). ¹³C NMR (75 MHz, D₂O) δ: 140.39, 125.17, 48.87, 45.03, 42.84, 33.09. Melting point: 239.4 ºC. Elemental analysis: Calculated: C 39.42%; H 7.71%; N 15.32% Experimental: C 39.35%; H 4.83%; N 14.30%.

***1-{4-pyridyl)-2,5-diazahexane.* (3).** C₉H₁₈N₃Cl₃ (Molecular weight = 294.60 g/mol). Yield: 70.56 %. ¹H NMR (300 Hz),: δ_{H} (ppm): 2.81 (s, 3H), 3.49 - 3.54 (m, 2H), 3.62 - 3.67 (m, 2H), 4.68 (s, 2H), 8.17 (d, *J* = 6.2Hz, 2H), 8.89 (d, *J* = 6.2Hz, 2H). ¹³C NMR (75 MHz, D₂O) δ: 151.02, 142.38, 127.14, 49.74, 44.37, 43.49, 33.22. Melting point: 220.5 ºC. Elemental analysis: Calculated: C 53.49%; H 6.59%; N 14.39% Experimental: C 53.49%; H 6.59%; N 14.39%.

***1-(2-pyridyl)-5-methyl-2,5-diazahexane.* (4)** C₁₀H₂₀N₃Cl₃ · 0.5 H₂O (Molecular weight = 297.65 g/mol), Yield: 65.3 %. ¹H NMR (300 Hz, ): δ_{H} (ppm : 3.00 (s, 6H), 3.58 (s, 4H), 4.49 (s, 2H), 7.63 - 7.71 (m, 2H), 8.13 (t, *J* = 7.8Hz, 1H), 8.67 (d, *J* = 5.3Hz, 1H). ¹³C NMR (75 MHz, ) δ: 147.43, 145.53, 143.95, 126.46, 126.30, 52.78, 49.34, 43.31, 42.04. Melting point: 213.7 ºC. Elemental analysis: Calculated: C 40.35%; H 7.11%; N 14.12% Experimental: C 40.73%; H 7.54%; N 14.41%.

***1-{3-pyridyl}-5-methyl-2,5-diazahexane.* (5)** C₁₀H₂₀N₃Cl₃ (Molecular weight = 288.64 g/mol), Yield: 64.15 %. ¹H NMR (300 Hz),: δ_{H} (ppm): 3.01 (s, 6H), 3.57 - 3.68 (m, 4H), 4.57 (s, 2H), 8.09 (d, *J* = 8.2Hz, *J* = 5.7Hz, 1H), 8.65 (d, *J* = 8.1Hz, 1H), 8.88 (d, *J* = 5.7Hz, 1H), 8.95 (s, 1H). ¹³C NMR (75 MHz, D₂O) δ: 148.00, 142.90, 142.79, 130.77, 127.89, 52.32, 47.83, 43.40, 41.99. Melting point: 218.9 ºC. Elemental analysis: Calculated: C 41.61%; H 6.98%; N 14.56% Experimental: C 41.82%; H 7.01%; N 14.45%.

***1-(4-pyridyl)-5-methyl-2,5-diazahexane.* (6)** C₁₀H₂₀N₃Cl₃ (Molecular weight = 288.64 g/mol). Yield: 67.8%, ¹H NMR (300 Hz, D₂O): δ_{H} (ppm): 3.02 (s, 6H), 3.59 - 3.70 (m, 4H), 4.64 (s, 2 H), 8.16 (d, *J* = 6.71Hz, 2H), 8.90 (d, *J* = 6.8Hz, 2H). ¹³C NMR (75 M Hz), δ 151.16, 142.20, 127.28, 52.44, 49.84, 43.40, 42.31. Melting point: 210.0 ºC. Elemental analysis: Calculated: C 41.61%; H 6.98%; N 14.56% Experimental: C 41.30%; H 6.83%; N 14.67%.

***1-(2-quinolylj-5-methyl-2,5-diazahexane.* (7)** C₁₄H₂₂N₃Cl₃ (Molecular weight = 338.70 g/mol), Yield: 66.25 %. ¹H NMR (300 Hz, D₂O): δ_{H} (ppm): 3.02 (s, 6H), 3.62 - 3.72 (m, 4H), 4.43 (s, 2H), 7.74 (d, *J=* 8.5Hz, 1H), 7.80 (t, *J=* 7.7Hz, 1H), 7.98 (t, *J* = 8.6Hz, 1H), 8.15 (t, *J* = 9.2Hz, 1H), 8.68 (d, *J* = 8.6Hz, 1H). ¹³C NMR (75 MHz, ) δ_{H}: 150.96, 143.66, 141.91, 132.49, 128.54, 128.01, 125.25, 120.46, 53.06, 50.58, 43.26, 42.01. Melting point: 211.1ºC. Elemental analysis: Calculated: C 49.65%; H 6.55%; N 12.41% Experimental: C 49.81%; H 6.37%; N 12.53%.

***1-{4-quinolyl)-5-methyl-2, 5-diazahexane.* (8)** C₁₄H₂₂N₃Cl₃ (Molecular weight = 338.70 g/mol), Yield: 66.62%, ¹H NMR (300 Hz,): δ_{H} (ppm): 3.02 (s, 6H), 3.59 - 3.68 (m, 4H), 5.04 (s, 2H), 8.06 (t, *J* = 7.7Hz, H), 8.12 (d, *J* = 5.7Hz, H) 8.21 (t, *J* = 7.8Hz, H), 8.32 (d, *J* = 8.6Hz, H), 8.43 (d, *J=* 8.6Hz, H), 9.17 (d, *J=* 5.6Hz, H). ¹³C NMR (75 MHz, D₂O) δ: 144.30, 138.02, 135.19, 130.85, 126.74, 124.13, 121.83, 120.71, 52.87, 47.63, 43.34, 42.60. Melting point: 211.1 ºC. Elemental analysis: Calculated: C 49.65%; H 6.55%; N 12.41% Experimental: C 49.55%; H 6.51%; N 12.56%.

***1-{2-quinolyl)-2,5-diazahexane.* (9).** C₁₃H₁₉N₃Cl₂ · 2H₂O (Molecular weight = 291.82 g/mol). Yield: 52.76 %. ¹H NMR (300 Hz),: δ_{H} (ppm): 2.85 (s, 3H), 3.55 - 3.63 (m, 4H), 4.69 (s, 2H), 7.63 (d, *J* = 8.5Hz, 1H), 7.75 (t, *J* = 7.5Hz, 1H), 7.93 (t, *J* = 7.5Hz, 1H), 8.06 - 8.15 (m, 2H), 8.55 (d, *J* = 8.5Hz, 1H). ¹³C NMR (75 MHz, ) δ: 151.63, 145.57, 139.65, 131.24, 128.29, 127.80, 126.99, 120.09,51.20, 44.83, 43.05, 33.10. Melting point: 215.8 ºC, Elemental Analysis: Calculated: C 36.94%; H 6.88%; N 14.35% Experimental: C 37.80%; H 6.72%; N 14.03%.

***1-(4-quinolyl}-2,5-diazahexane.* (10).** C₁₃H₂₀N₃Cl₃ · 2H₂O (Molecular weight = 360.71 g/mol). Yield: 64.34 %. ¹H NMR (300 Hz),: δ_{H} (ppm): 2.85 (s, 3H), 3.50 - 3.66 (m, 4H), 5.04 (s, 2H), 8.01 - 8.08, (m, 2H), 8.19 (t, *J* = 7.8Hz, 1H), 8.31 (d, *J* = 8.8Hz, 1H), 8.40 (d, *J* = 8.7Hz, 1H), 9.15 (d, *J* = 5.6Hz, 1H). ¹³C NMR (75 M Hz), δ: 148.56, 144.57, 138.45, 134.99, 130.74, 126.66, 124.05, 122.16, 120.67, 47.50, 44.71, 43.80, 33.20. Melting point: 214.8 ºC. Elemental analysis: Calculated: C 43.44%; H 6.73%; N 11.69% Experimental: C 43.29%; H 6.70%; N 11.65%.

**FEM measurements.** Potentiometric titrations were conducted at 298.1 ± 0.1 K using 0.15 M NaCl as the support electrolyte. The experimental procedure (burette, potentiometer, cell, stirrer, microcomputer, etc.) has been fully described elsewhere (E. Garcia-Spain, M.-J. Ballester, F. Lloret, J.M. Moratal, J. Faus and A. Bianchi, J. Chem. Soc. Dalton Trans. 1988, 101-104). The FEM data acquisition was performed with the PASAT software (M. Fontanelli and M. Micheloni, Program for the automatic control of the microburette and the acquisition of the electromotor force readings (PASAT). Minutes of the ^{1st} Spanish-Italian Congress of Thermodynamics of Metal Complexes, Peñíscola, Castellón, Spain, 1990). The reference electrode was an Ag/AgCI electrode in saturated KCI solution. The glass electrode was calibrated as a hydrogen ion concentration probe by titration of previously standardised amounts of HCl with CO₂ free NaOH solutions and the equivalent point determined by Gran's method ((a) G. Gran, Analyst 1952, 77, 661 -671; (b) F.J. Rossotti and H. Rossotti, J. Chem. Educ. 1965, 42, 375-378) providing the standard potential, Eº', and the ionic product of water (pKw = 13.73 (1)).

Hyperquad software was used to calculate protonation and stability constants (P. Gans, A. Sabatini and A. Vacca, Talanta 1996, 43, 1739-1753). The pH range investigated was 2.5-11.0 and the concentration of the ligands varied from 1×10⁻³ to 5×10⁻³ M. The different titration curves for each system (at least two) were treated as a single set or as separate curves without significant variations in the values of the stability constants. Finally, the data sets were fused and treated simultaneously to give the final protonation constants (Table 1). For compounds 1, 7 and 9, two protonation constants have been detected in the pH range studied with values ranging from 5.51 to 9.68 logarithmic units. For the remaining compounds, three protonation constants have been measured with values ranging from 10.08 to 2.25 logarithmic units. All compounds have charge +1 at the physiological pH value of 7.4. These compound characterisation results do not make their administration to human beings or animals inadvisable.

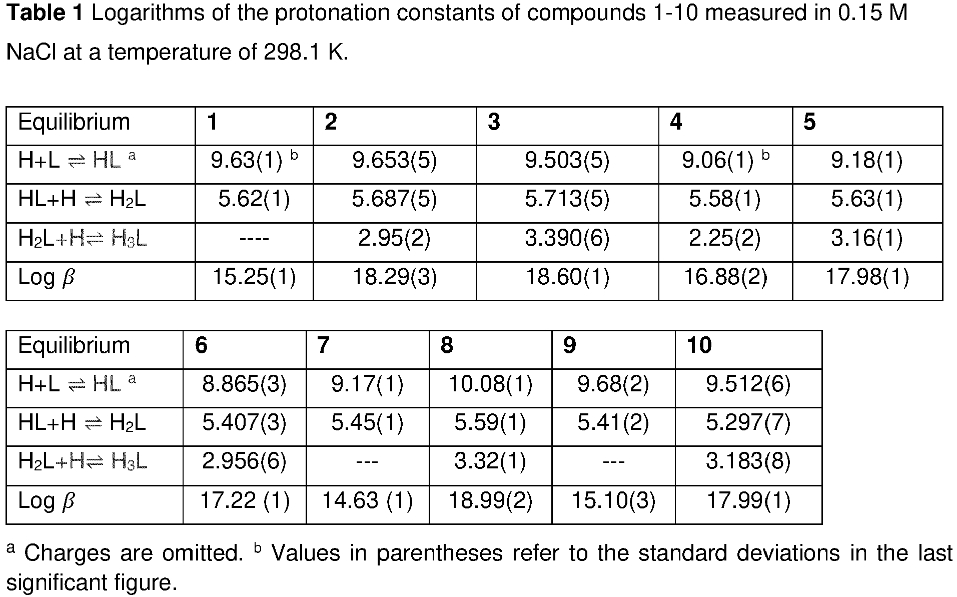

### Example 2.- In vitro activity evaluation. Toxicity and selectivity indices of compounds 1-10.

This Example describes the procedure for evaluating the *in vitro* activity of compounds 1-10 against extracellular (promastigotes) and intracellular (amastigotes) forms of *L. infantum species. L. braziliensis* and *L. donovani,* the J774.2 macrophage toxicity assessment procedure and the Selectivity Index calculations.

*Methodology:* Cultures of three Leishmania species, representative of the species causing each of the clinical forms of the disease, were used for these studies. Specifically, strains MCAN/ES/2001/UCM-1 O of *L. infantum,* isolated and characterised in Madrid from an infected dog; MHOM/BR/1975/M2904 of *L. braziliensis,* isolated in Brazil in a human case, and LCR-L 133 LRC of *L. donovani.* isolated in Jerusalem, Israel from a case of kala-azar from an Ethiopian patient (WHO Technical Reports Series 949. Geneva: WHO. March. The cultures of parasites come from an own collection which was prepared from a biobank project (Biobank and Characterisation Unit of Strains and Species of Trypanosomatids, Responsible for Human, Animal and Plant Pathologies, Cgl-2008-03687-E/Bos.), and are available on request.

The promastigote forms of the three species studied were cultured *in vitro* in sterility in single-phase MTL culture medium (Medium Trypanosomes Liquid) made from Hank's Balanced Salts (Sigma-Aldrich, H6136) enriched with 10% (v/v) of Fetal Bovine Serum (SBF-1) inactivated at 56°C/ 30 minutes. The parasite inoculum to initiate the culture was 5 × 10⁴ cells/ml in 5 ml medium in 25 cm² Falcon^{®} plastic flasks and kept in an oven at 28°C. The cultures were performed routinely, achieving exponential growth of the flagellates until obtaining the cell mass necessary for the subsequent studies. Both promastigote forms and amastigote forms were used to conduct the assays.

The promastigote forms of the three *Leishmania* strains, cultured in the manner described above, were harvested in their exponential growth phase by centrifugation at 400 g for 10 min. The number of parasites was counted in a Neubauer Haemocytometer Counting Chamber and were inoculated in a 24-well plate at a concentration of 5 ×10⁴ parasites/well.

The compounds to be assayed were dissolved in DMSO at a concentration of 0.01% (v/v), concentration at which this solvent is not toxic nor has any effect on the growth of parasites. The compounds were added to the culture medium at final concentrations of 100, 50, 25 and 12.5 µM. The effect of each compound on the growth of the promastigote forms at the different concentrations assayed was evaluated at 72 h using a Neubauer Haemocytometer Counting Chamber and the leishmanicidal effect was expressed as the IC₅₀ (concentration required to give an inhibition of 50%, calculated by the analysis of the linear regression of the Kc (gradient of the line that adjusts to the concentrations) at the concentrations assayed) [Sánchez-Moreno. M. et al. Med. Chem. 2011. 54. 970-979].

For the study of the *in vitro* effect on intracellular forms of *Leishmania,* the experimental model designed by the group of the present inventors was used [Sánchez-Moreno. M. et al. Med. Chem. 2012, 55, 9900-9913; Sánchez-Moreno. M. et al. J. Antimicrob. Chemother. 2012, 67, 387-397]. The macrophages were detached from the culture flask where they were attached by dry blows. To do this, it was incubated in cold for 5 minutes and after that the flask was hit with the palm of the hand until the cells were detached from the flask. Then, they were passed to a 25 ml capacity steriling conical bottom flask (Deltalab) to centrifuge them at 120 g for 5 minutes, removing the supernatant and were counted in Neubauer's chamber. Cells were suspended in RPMI medium at a concentration of 1×10⁴ cells/well, then cultured in 24-well plates into which a 12 mm round slide had previously been placed into each well. For adherence, the cells were left 24 h at 37 °C in 5% CO₂. Subsequently, the cells were infected *in vitro* by placing *Leishmania* cells at the rate of 10 parasites/macrophage according to [Sánchez-Moreno. M et al. Med. Chem. 2011, 54, 970-979]. During the next 24 hours, the parasites infect the cells, after that time, the culture medium was removed to replace it by fresh medium with the products to be assayed at concentrations of 100, 50, 25 and 12.5 µM in order to calculate the IC₅₀. The products were allowed to act for 72 hours of incubation.

After that time, the crystals were removed and fixed with methanol on a slide and allowed to dry, after which DPX microscopy mounting medium (Panreac^{®}) was added to it. Once the preparations had been mounted, they were dyed with Giemsa by diluting Azur-Eosin-Methylene Blue solution according to Giemsa DC (Code 251338) with Sorensen buffer (1:10 dilution). The preparations were covered and allowed to colour for 20 minutes, after which they were washed with distilled water and then allowed to drain and dry vertically. Finally, the amastigote forms were counted with the aim of immersion in a total of 200 cells.

Macrophage toxicity: this study used the experimental model designed by the group of inventors [I. Ramírez-Macías. et. al. J. Antimicrob. Chemother. 2011, 66(4): 813-819] wherein flow cytometry is used. Briefly, macrophages from line J774.2 (ECACC number 91051511, obtained from a BALB/c female rat tumour in 1968) were deposited in a steriling tube and centrifuged at 120 g for 5 minutes. The supernatant was discarded and the cells were resuspended in MEM + Glutamine medium with 20% SBF (fetal bovine serum). 1×10⁴ cells per well were then deposited from a 24-well titration plate and incubated for 24-48 h at 37°C in a humid atmosphere enriched with 5% CO₂ to fix them. After that time, the culture media was removed and fresh media was added with the products to be assayed at concentrations of 100, 50, 25 and 12.5 µM. After 72 h of incubation, the samples were prepared for reading on a flow cytometer. Specifically, 100 µl of propidium iodide solution (100 µg/ml) was added to each well and incubated for 10 min at 28 °C in the dark. Subsequently, 100 µl/well of fluorescein diacetate (FDA) (Sigma Chemical Co) in solution (100 ng/mL) was added and incubation was repeated under the same conditions for 10 minutes. Cells were recovered by centrifugation at 400 g for 10 minutes and washing the precipitate with PBS. Finally, the results were analysed in a FACS Vantage flow cytometer (Becton Dickinson) taking into account that the cells with the plasma membrane intact emit green colour as the esterases act on the FDA, while the cells that have lost the integrity of the membrane and are not viable, emit in the range close to 580 nm by penetrating the propidium iodide by passive diffusion and specifically binding to the nucleic acids thereof. Thus, to calculate the percentage of viability, the number of dead cells compared to controls was taken into account. The IC₅₀ (concentration needed to obtain 50% inhibition against macrophage cells after 72 h of culture) was calculated using the analysis of the linear regression of Kc at the assayed concentrations.

The results obtained, which correspond to five independent experiments, are shown in Table 2 below:

**Table 2. Activity and toxicity found in compounds 1-10 against extracellular and intracellular forms of Leishmania**

| Compound | IC₅₀ activity (µM)^{a} | | | | | | Macrophage toxicity IC₅₀ (µM)^{b} |
|---|---|---|---|---|---|---|---|
| | *L. infantum* | | *L. braziliesis* | | *L. donovani* | | |
| | Promastigote forms | Amastigote forms | Promastigote forms | Amastigote forms | Promastigote forms | Amastigote forms | |
| Glucantime | 18.0±3.1 | 24.2±2.6 | 18.0±3.1 | 24.2±2.6 | 18.0±3.1 | 24.2±2.6 | 15.2±1.0 |
| 1 | 0.6±0.2 | 0.7±0.1 | 7.4±0.6 | 5.7±0.3 | 7.0±0.5 | 8.9±0.9 | 13.7±3.1 |
| 2 | 6.4±0.6 | 8.4±0.5 | 4.1±0.2 | 15.8±0.5 | 11.7±0.8 | 14.4±0.1 | 310.3±11.5 |
| 3 | 12.6±2.1 | 13.9±1.0 | 14.3±1.0 | 27.9±1.2 | 18.4±0.8 | 5.1±0.3 | 222.7±17.3 |
| 4 | 28.6±2.5 | 33.6±1.4 | 37.3±0.9 | 30.8±1.3 | 34.8±1.5 | 38.8±1.4 | 1241.7±42.7 |
| 5 | 9.2±0.7 | 10.0±0.8 | 16.7±0.5 | 20.0±2.0 | 21.7±1.6 | 17.6±0.7 | 230.4±16.9 |

| | *L. infantum* | | *L. braziliensis* | | *L. donovani* | | |
|---|---|---|---|---|---|---|---|
| Promastigote forms | Amastigote forms | Promastigote forms | Amastigote forms | Promastigote forms | Amastigote forms | | |
| 6 | 63.3±1.5 | 23.6±1.7 | 52.5±5.1 | 29.7±2.5 | 67.9±5.3 | 11.5±1.3 | 496.9±21.5 |
| 7 | 84.4±5.1 | 49.7±3.6 | 105.3±11.6 | 38.9±2.7 | 56.9±2.7 | 38.9±3.2 | 442.4±31.6 |
| 8 | 14.1±0.6 | 11.5±0.8 | 13.8±0.7 | 16.8±1.1 | 12.8±0.6 | 10.6±0.6 | 22.5±0.6 |
| 9 | 15.6±0.8 | 14.3±0.6 | 28.0±0.3 | 26.5±0.9 | 27.5±0.4 | 31.9±1.1 | 353.9±31.7 |
| 10 | 12.5±1.1 | 18.7±1.5 | 18.4±1.1 | 21.7±2.3 | 24.1±0.6 | 26.8±2.1 | 164.3±10.0 |

| | | | | | | | |
|---|---|---|---|---|---|---|---|
| ^{a} IC₅₀ = Concentration necessary to obtain 50% inhibition, calculated by linear regression analysis of the Kc value at the concentrations used of (12.5, 25, 50 and 100 µM). ^{b} Concentration necessary to obtain 50% inhibition against macrophage cells after 72 h of culture. | | | | | | | |

With the above results, the selectivity indices (SI) for extracellular and intracellular forms of *Leishmania* were calculated.

The results corresponding to the five independent experiments are shown in Table 3.

**Table 3.- Selectivity indices found for extracellular and intracellular forms of Leishmania**

| Compound | Selectivity Index (SI)^{c} | | | | | |
|---|---|---|---|---|---|---|
| | *L. infantum* | | *L. braziliensis* | | *L. donovani* | |
| | Promasti gate forms | Amastigo te forms | Promastig ote forms | Amastig ote forms | Promastig ote forms | Amastig ote forms |
| Glucantime | 0.8 | 0.6 | 0.6 | 0.6 | 0.7 | 0.6 |
| 1 | 23(28) | 20 (33) | 2(3) | 2 (4) | 2 (3) | 1(3) |
| 2 | 48(61) | 37 (62) | 76 (126) | 20 (33) | 26 (38) | 21 (36) |
| 3 | 18 (22) | 16 (27) | 16 (26) | 8 (13) | 12 (17) | 44 (73) |
| 4 | 43 (54) | 37 (62) | 33 (55) | 40 (67) | 36(51) | 32 (53) |
| 5 | 25(42) | 23 (38) | 14 (23) | 11(19) | 11(15) | 13(22) |
| 6 | 8(10) | 21 (35) | 9(16) | 17 (28) | 7(10) | 43 (72) |
| 7 | 5(7) | 9(15) | 4(7) | 11(19) | 8(11) | 11(19) |
| 8 | 2(2) | 2 (3) | 2 (3) | 1(2) | 2 (2) | 2 (3) |
| 9 | 23(28) | 25(41) | 13(21) | 13(22) | 13(19) | 11(18) |
| 10 | 13(16) | 9(15) | 9(15) | 8(13) | 7(10) | 6(10) |

| | | | | | | |
|---|---|---|---|---|---|---|
| ^{c} Selectivity index = IC₅₀ of macrophages / IC₅₀ of extracellular and intracellular forms of the different *Leishmania* species studied. In parentheses, the number of times the SI exceeds that of the reference drug | | | | | | |

As can be seen in the tables above, all compounds showed activity against the promastigote and amastigote forms of any of the three *Leishmania* strains included in the assay, activity that in most cases is even higher than that of the reference compound. It is very noteworthy that almost all the compounds of the present invention have a lower toxicity in macrophages than the reference compound, Glucantime^{®}, being very noteworthy that the data obtained with some of the compounds, such as 4, whose IC₅₀ in macrophages is almost two orders of magnitude lower than that of the reference compound, i.e. a concentration almost 2 orders of magnitude higher than compound 4 is needed to obtain the same toxicity as with the reference compound. Also very notable are the low toxicities of compounds 6 and 7, as well as those of compounds 9, 2 and 3. Compound 1 is the only one that has an IC₅₀ value in macrophages similar to that of the reference compound, slightly lower in the mean value, although practically the same if the oscillation range is considered.

On the other hand, the data of Table 3 should be highlighted, wherein it can be seen that all the compounds of the present invention have a selectivity index several times higher than that of the reference compound for all the strains assayed and in all their forms. The data of compound 4, whose selectivity rates are between 51 and 67 times higher than those of the reference compound, are again particularly noteworthy, depending on the strain and the form of the same with which the assay has been conducted. The data obtained with compound 2, which has selectivity indices similar to those of compound 4 in the assays carried out with the *L. infantum* strain, and which has a very high selectivity index of 126, with respect to the promastigote forms of the *L. braziliensis* strain used in the assay, are also very noteworthy. The values obtained for compounds 3 and 6 wherein it refers to the amastigote forms (the most important) of *L. donovani* are also high, as well as those obtained with compound 5 wherein it refers to both forms of the *L. infantum* strain, results that caused compound 5 to be included in the following experiments.

With these results, it was decided to continue the assays with compounds 2, 4 and 5.

Example 3.- Evaluation of infection rate and parasite growth in macrophage cultures infected with *Leishmania* strains (compounds 2, 4 and 5).

*Methodology:* Macrophages of the J774.2 line preserved in the laboratory of the present inventors by cryopreservation at -80°C and successive runs in MEM+Glutamine + 20% SBFI medium were used, which were deposited in a steriling tube and centrifuged at 100 g for 5 minutes. The supernatant was discarded and the cells were resuspended in medium corresponding to a final concentration of 1×10⁶cells/ml.

Subsequently, 10 µl of the cell suspension was deposited into each well of a 24-well culture plate each with a 10 mm diameter round slide. A final volume of 500 µl was completed with the culture medium and incubated for 24 h at 37°C in humid atmosphere with 5% CO₂.

Subsequently, the cells were infected with promastigote forms of the *Leishmania* species corresponding to each assay, at a 10:1 ratio of promastigotes per macrophage. Compounds 2 and 4 (and 5 for *L. infantum* only) were added to reach an IC₂₅ concentration just after infection and incubated for 12 h at 37°C in 5% CO₂. The non-phagocytic parasites and products were removed by washing and the infected cultures were maintained for 10 days in fresh medium added every 48 hours. The activity of the compounds was determined by the percentage of infected cells and the number of amastigotes per infected cell. Finally, the cultures were fixed and stained with methanol and Giemsa. The percentage of infected cells and the mean number of amastigotes per infected cell was determined by counting 200 cells under a microscope for 48 hours.

As can be seen in Figures 2, 3 and 4, both the infection rate values and the number of amastigotes per macrophage were, in all cases, lower than those obtained with the controls without compound and, even, those obtained with the reference drug. In all cases, the best results were obtained with compound 2, as it was the one that resulted in the lowest values over time. In the case of the *L. infantum* assays wherein the behaviour of each of the three compounds was assayed, compound 5 resulted in lower infection rates than compound 4 until 8 days had elapsed, while compound 4 resulted in a lower infection rate on day 10; when comparing the values of the number of amastigotes per macrophage in those same assays, the results obtained with compound 4 were better (lower) than those obtained with compound 5.

Example 4.- Description of the ability of compounds 2, 4 and 5 to inhibit the activity of the FeSOD enzyme of the promastigotes of the three *Leishmania* species in relation to the CuZnSOD of human erythrocytes.

*Methodology:* Compounds that were selected from the leishmanicidal activity shown in previous experiments were evaluated as inhibitors of FeSOD enzyme activity, which is unique to the kinetoplastid parasites to which *Leishmania belongs.* Being exclusive, it is absent in the vertebrate host, since these usually have an analogous form of that enzyme, but complexed to a copper or zinc atom (Cu/ZnSOD). For this reason, this enzyme is a therapeutic target for the design of new drugs, since the capture of the metal ion by complexing mechanisms can inactivate the enzyme and compromise the survival of the parasite with the appearance of free radicals.

To conduct this assay, the Cu/Zn-superoxide dismutase of human red blood cells from Boehringer Mannheim was used, whilst the coenzymes and substrates were obtained from Sigma-Aldrich.

Inhibition caused by the novel compounds on FeSOD/ Cu-Zn SOD activity was quantified on the spectrophotometer at 560 nm according to the technique described by [W F. Beyer. I. Fridovich. Anal Biochem. 1987. 161:559-66] based on the determination of the reduction of NBT (nitro blue tetrazolium) by superoxide ions and which the group of the present inventors has developed for the assay in trypanosomatides, being a routine technique in the laboratory of said group [F. Luque. et. al. Comp. Biochem. Physiol. C Toxicol. Pharmacol. 2000. 126:39-44; M. Sanchez-Moreno. Med. Chem. 2011. 54. 970-9; I. Ramírez-Macías. et. al. J. Antimicrob. Chemother 2011. 66(4):913-9].

Briefly, the cultured parasites as described in Example 1 underwent centrifugation, the pellet was resuspended (0.5 - 0.6 g/ml) in 3 ml of STE buffer (0.25 M. Tris-HCl 25 mM sucrose. 1 M EDTA. pH 7.8) and underwent 3 cycles of sonication from 30 s at 60 V. Then, it was centrifuged at 1500 g for 10 min at 4 °C and the pellet was washed 3 times with ice-cooled STE buffer. This fraction was centrifuged (2500 *g* for 10 min at 4 °C) and the supernatant was subsequently collected. Protein concentrations were determined by means of the Bradford method (Sigma-Aldrich) using bovine serum albumin as standard. To determine the activity of the enzyme superoxide dismutase FeSOD, located in the *stock* solution (phosphate buffer 50 mM. pH 7.8. 54 ml, L-methionine 3 ml, NBT 2 ml, Triton X-100 1.5 ml) one of the compounds to be assayed and riboflavin were added to each cuvette. A first measurement of the absorbance at 560 nm was made on a spectrophotometer and, after 10 minutes under light and under stirring conditions, the absorbance was re-determined. One unit was considered to be the amount of enzyme needed to inhibit the rate of NBT reduction by 50%.

The results obtained are shown in the graphs of Fig. 5. The graphs also indicate the concentration required to give 50% inhibition with each compound.

As can be seen, the concentration necessary for any of compounds 2, 4 and 5 to result in 50% inhibition of the FeSOD enzyme of the promastigote forms of *L. infantum* is less than that necessary to achieve the same percentage of inhibition of the CuZnSOD enzyme of human erythrocytes. This fact is also observed when the FeSOD inhibition data of the amastigote forms of *L. donovani* by compounds 2 and 4 were compared, and in the FeSOD inhibition of the promastigote forms of *L. braziliensis* by compound 4, so that in almost all cases the assayed compounds are capable of inhibiting the parasite FeSOD enzyme with an IC₅₀ value lower than that necessary for the enzyme of human erythrocytes.

Example 5.- Study of the alteration of the glycolytic pathway of the parasites by the action of the new compounds.

*Methodology:* Promastigotes from the three species studied were used for this study. They were left to grow in a culture flask and subsequently transferred to a 25 ml capacity conical bottom flask (steriling) so that there were 5×10⁴ parasites/ml in each one. The amount of compound needed to reach IC₂₅ of each product was added and completed with fresh culture medium to reach a final volume of 3 ml and incubated at 28°C for 72 h. After that time, the flasks were centrifuged at 400 g for 10 minutes, and 2 ml of supernatant was transferred to Eppendorf^{®} tubes. Next, they were analysed using proton nuclear resonance and the spectra were analysed with the Mestre Nova^{®} software to obtain the metabolite production and excretion graphs. From these graphs, it can be inferred which enzyme has seen its activity altered by action of the compounds, depending on the increase or decrease of the metabolite produced by it.

Both the decrease and increase in the metabolites analysed can be positive effects, indicating a malfunctioning of the metabolic pathway. An increase in one metabolite whilst the others fall indicates that the reaction that produces the metabolite is being repeated and the rest of the reactions do not occur, resulting in a blockage of the metabolism.

The results obtained are shown in Fig. 6.

As can be seen, all compounds alter the metabolism of the three species used in this study, although compound 2 only produces a certain increase of succinate against *L. braziliensis* and practically does not alter the other metabolites analysed. In contrast, the increase in succinate production caused by compound 4 in both *L. infantum* and *L. braziliensis* (wherein it produces a decrease of the remaining metabolites analysed), and by compound 2 in *L. donovani* is noteworthy.

In this case, the increase of succinate and the decrease of the other metabolites in *L. infantum* and *L. braziliensis* indicate that the glucose is not being processed correctly (the parasite is not obtaining energy), since the reaction that the succinate uses as a substrate is not taking place: therefore its excretion increases. In the case of the effects of compound 2 on *L. donovani,* they can be interpreted as a metabolic acceleration, since all the metabolites analysed increase, which can lead to depletion of the glucose of the medium.

Example 6.- Analysis of the alteration capacity of the mitochondrial membrane potential of the new compounds by rhodamine staining and by flow cytometry.

*Methodology:* Similar to the assay of Example 5, an amount of 5x10 4 parasites/ml of *Leishmania donovani* was re-treated with the IC₂₅ of compounds 2 and 4, this time in a final volume in the 5 ml steriling conical bottom flasks. Again, after 72 h of incubation under the same conditions as described in Example 5, the flasks were centrifuged at 400 g for 10 minutes and the pellet was washed by resuspending with PBS three times. The pellet was resuspended with 0.5 mL of PBS with 10 µg/mL rhodamine after the last wash. A cytometry analysis was performed after 20 minutes of action of the rhodamine.

The results obtained are shown in Fig. 7, wherein it can be seen that the presence of compounds 2 or 4 results in only a slight depolarisation of the parasite's mitochondrial membrane, and therefore it does not seem that such depolarisation can be the main cause for which said compounds are effective. Based on the results of the SOD inhibition experiment (although without wanting to be limited by any hypothesis) it is most likely that the compounds are active due to their ability to inhibit the enzymatic activity first, and second, by altering the metabolism. Furthermore, metabolic disturbances can sometimes be due to SOD inhibition.

## Claims

1. A compound of Formula (I) 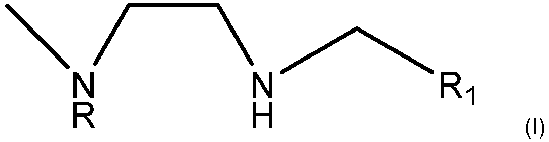 where
R is selected from H or CH₃; and
R₁ is selected from the group of 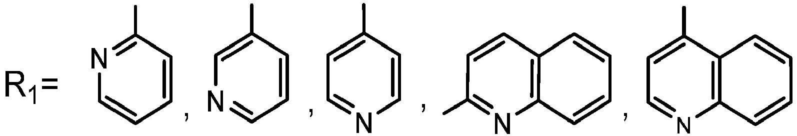
or a pharmaceutically acceptable salt or solvate thereof,
for its use in the treatment of leishmaniasis.

2. The compound for its use according to claim 1, wherein the compound is selected from the group of compounds of formulas: 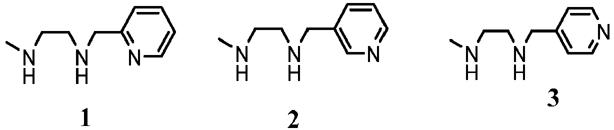 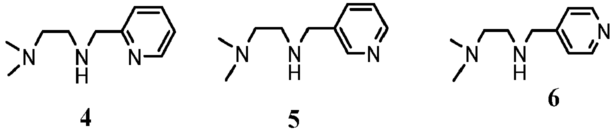 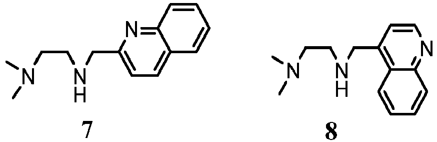 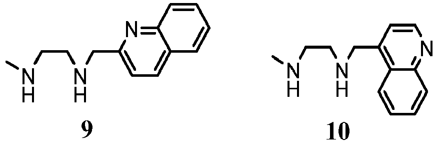 or the pharmaceutically acceptable salts and solvates thereof.

3. The compound for its use according to claim 2, wherein the compound is selected from the group of compounds 3 (1-{4-pyridyl) -2,5-diazahexane), 4 (1-(2-pyridyl) -5-methyl-2,5-diazahexane), 5 (1-{3-pyridyl} -5-methyl-2,5-diazahexane), 6 (1-(4-pyridyl) -5-methyl-2,5-diazahexane), 7 (1-(2-quinolyl} -5-methyl-2,5-diazahexane) and 9 (1-{2-quinolyl) -2,5-diazahexane) and the pharmaceutically acceptable salts and solvates thereof.

4. The compound for its use according to claim 2, wherein the compound is selected from the group of compounds 2 (1-{3-pyridyl) -2,5-diazahexane), 4 (1-(2-pyridyl)-5-methyl-2,5-diazahexane) and 5 (1-{3-pyridyl}-5-methyl-2,5-diazahexane) and the pharmaceutically acceptable salts and solvates thereof.

5. The compound for its use according to claim 2, wherein the compound is compound 5 (1-{3-pyridyl} -5-methyl-2,5-diazahexane) or a pharmaceutically acceptable salt or solvate thereof.

6. The compound for its use according to any of claims 1-5, wherein the leishmaniasis is caused by a species of the genus *Leishmania* selected from *L. tropica, L. major, L. aethiopica, L. braziliensis, L. panamensis*/*L. guyanensis, L. shawi, L. peruviana, L. mexicana, L. amazonensis, L. venezuelensis, L. lainsoni, L. naiffi, L. linderbergi, L. infantum and L. donovani.*

7. The compound for its use according to claim 4, wherein the leishmaniasis is caused by the species *L. infantum* and the compound is selected from the group of compounds 2 (1-{3-pyridyl)-2,5-diazahexane), 4 (1-(2-pyridyl) -5-methyl-2,5-diazahexane) and 5 (1-{3-pyridyl} -5-methyl-2,5-diazahexane) and the pharmaceutically acceptable salts and solvates thereof.

8. The compound for its use according to claim 4, wherein the leishmaniasis is caused by the species *L. braziliensis* and the compound is selected from the group of compounds 2 (1-{3-pyridyl)-2,5-diazahexane) and 4 (1-(2-pyridyl) -5-methyl-2,5-diazahexane) and the pharmaceutically acceptable salts and solvates thereof.

9. The compound for its use according to claim 4, wherein the leishmaniasis is caused by the species *L. donovani* and the compound is selected from the group of compounds 2 (1-{3-pyridyl)-2,5-diazahexane) and 4 (1-(2-pyridyl) -5-methyl-2,5-diazahexane) and the pharmaceutically acceptable salts and solvates thereof.

10. The compound for its use according to any one of claims 1-4, wherein the compound is for its use in the treatment of leishmaniasis in a mammal selected from the group of the dog, wolf, fox, cat, horse, mule, donkey, sheep, goat, cow and human being.

11. The compound for its use according to claim 10, wherein the mammal is a human being and the leishmaniasis is selected from cutaneous leishmaniasis, visceral leishmaniasis, and mucocutaneous leishmaniasis.

12. The compound for its use according to claim 11, wherein the leishmaniasis is cutaneous leishmaniasis and the compound is selected from the group of compounds 2 (1-{3-pyridyl)-2,5-diazahexane), 4 (1-(2-pyridyl)-5-methyl-2,5-diazahexane) and 5 (1-{3-pyridyl} -5-methyl-2,5-diazahexane) and the pharmaceutically acceptable salts and solvates thereof.

13. The compound for its use according to claim 11, wherein the leishmaniasis is mucocutaneous leishmaniasis and the compound is selected from the group of compounds 2 (1-{3-pyridyl)-2,5-diazahexane) and 4 (1-(2-pyridyl) -5-methyl-2,5-diazahexane), and the pharmaceutically acceptable salts and solvates thereof.

14. The compound for its use according to claim 11, wherein the leishmaniasis is visceral leishmaniasis and the compound is selected from the group of compounds 2 (1-{3-pyridyl)-2,5-diazahexane) and 4 (1-(2-pyridyl) -5-methyl-2,5-diazahexane), and the pharmaceutically acceptable salts and solvates thereof.

15. The compound for its use according to claim 10, wherein the mammal is a dog and the compound is selected from the group of 2 (1-{3-pyridyl)-2,5-diazahexane), 4 (1-(2-pyridyl)-5-methyl-2,5-diazahexane), and 5 (1-{3-pyridyl} -5-methyl-2,5-diazahexane), and the pharmaceutically acceptable salts and solvates thereof.

16. A pharmaceutical or veterinary composition comprising at least one compound of Formula (I) 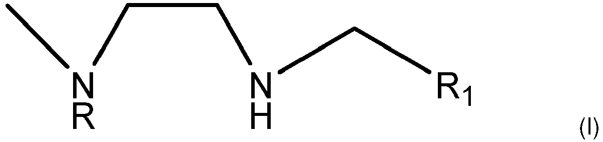 or a pharmaceutically acceptable salt or solvate thereof, wherein
R is selected from H or CH₃, and
R₁ is selected from the group of 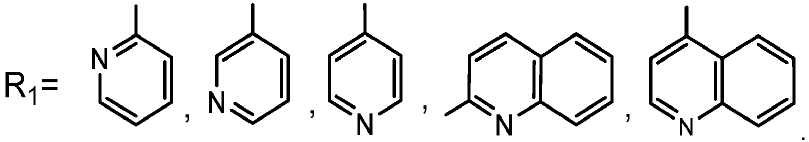
and optionally comprising one or more pharmaceutically acceptable excipients and/or vehicles, for its use in the treatment of leishmaniasis.

17. The composition for its use according to claim 16, wherein the compound of Formula (I) is selected from the group of compounds of the following formulas 1 to 10: 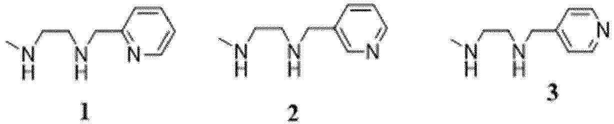 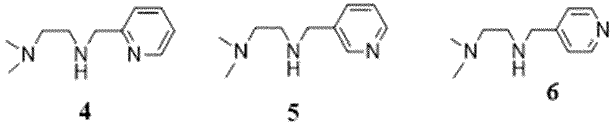 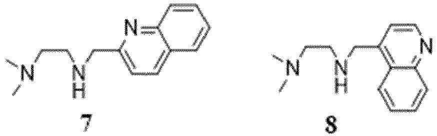 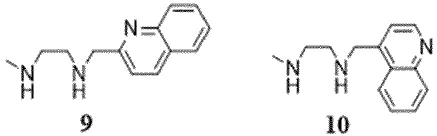 and pharmaceutically acceptable salts and solvates thereof.

18. The composition for its use according to claim 16 or 17, comprising more than one compound of Formula (I) as defined in claim 16 or claim 17, or more than one solvate or salt thereof.

19. A combined pharmaceutical or veterinary preparation comprising:
a) at least one compound of Formula (I) 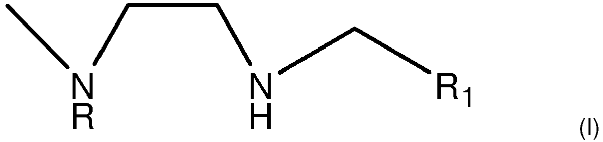 or a pharmaceutically acceptable salt or solvate thereof, wherein
R is selected from H or CH₃, and
R₁ is selected from the group of 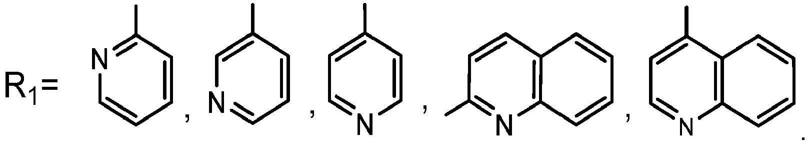
or combinations thereof,
and additionally
b) at least one antiparasitic agent with activity against a parasite of the family *Trypanosomatidae,* wherein the additional antiparasitic agent is different from the compounds defined in a),
for its use in the treatment of leishmaniasis.

20. The combined pharmaceutical or veterinary preparation for its use according to claim 19, wherein the additional antiparasitic agent is selected from the group of:
i. Meglumine antimoniate,
ii. Sodium stibogluconate,
iii. Benznidazole,
iv. a compound selected from the group of the following compounds of formulas (II)sc, (III)sc, (IV)sc, (V)sc, (VI)sc, (VII)sc, (VIII)sc, (IX)sc, (X)sc, (Xl)sc, (XII)sc, (XIII)sc, (XIV)sc, (XV)sc, (XVI)sc, (XVII)sc, and (XVIII)sc 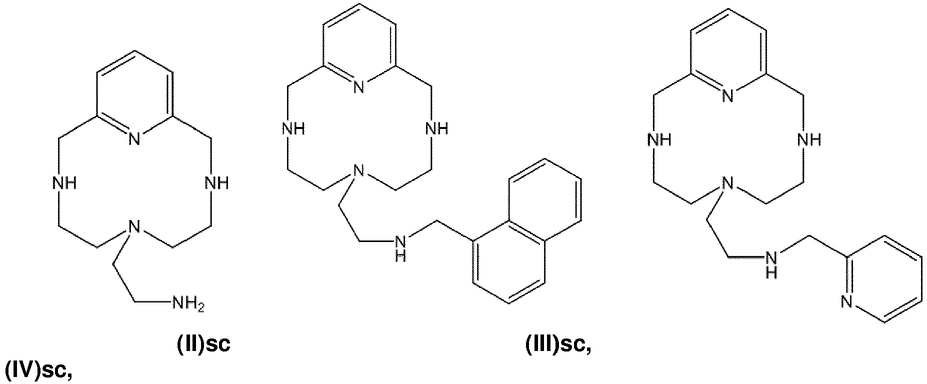 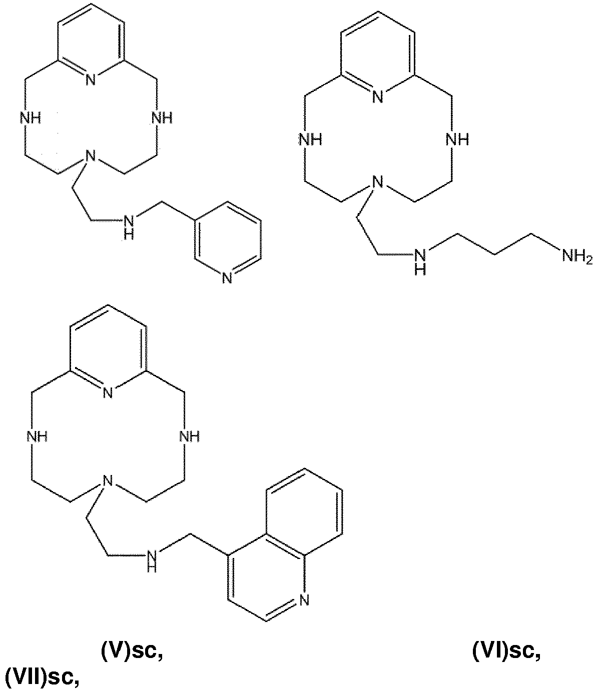 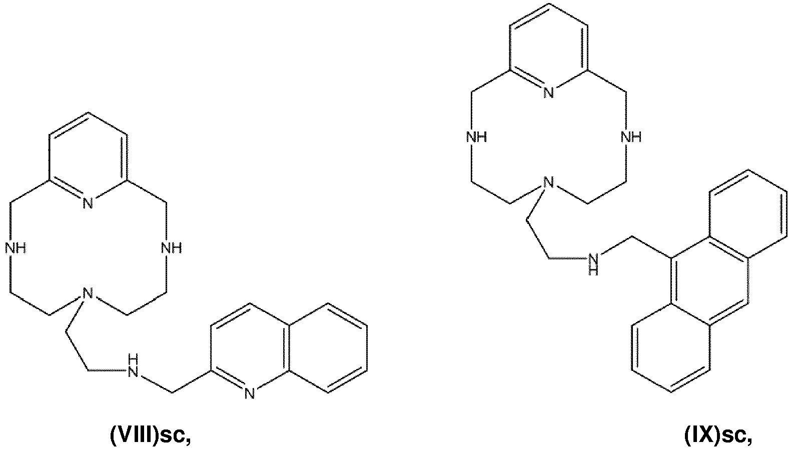 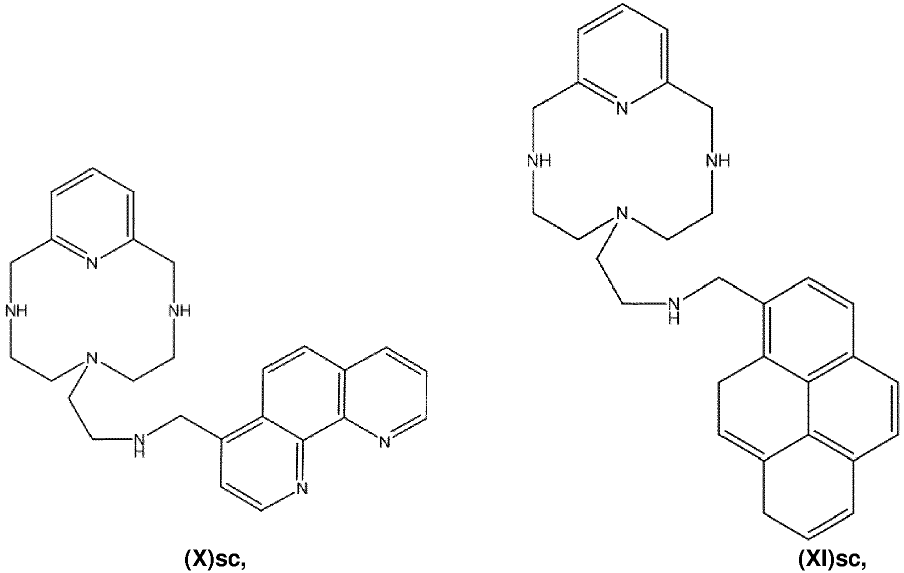 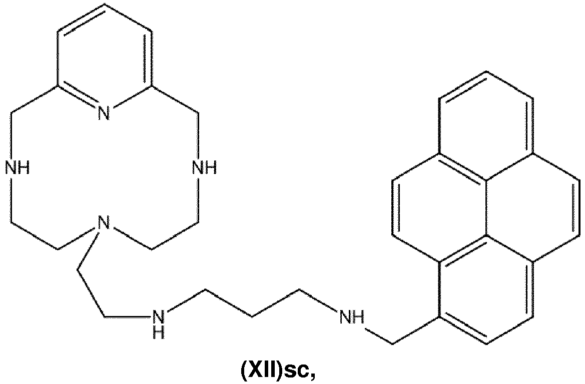 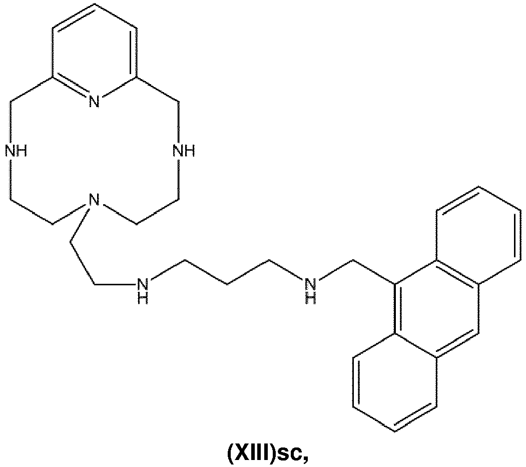 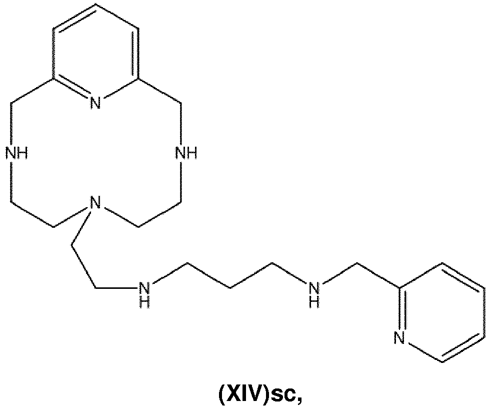 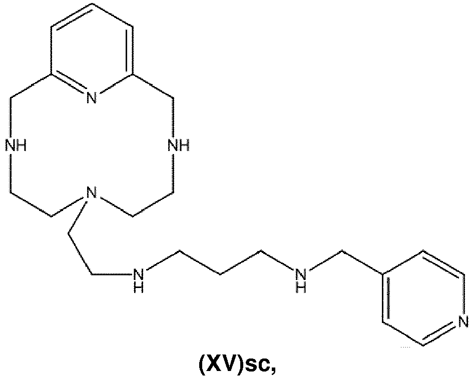 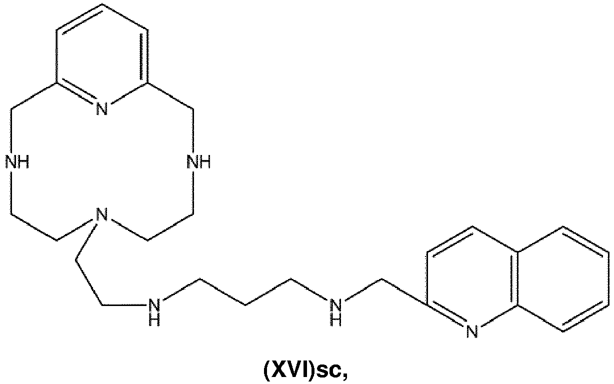 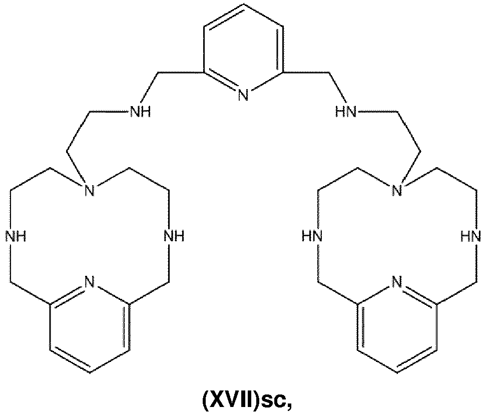 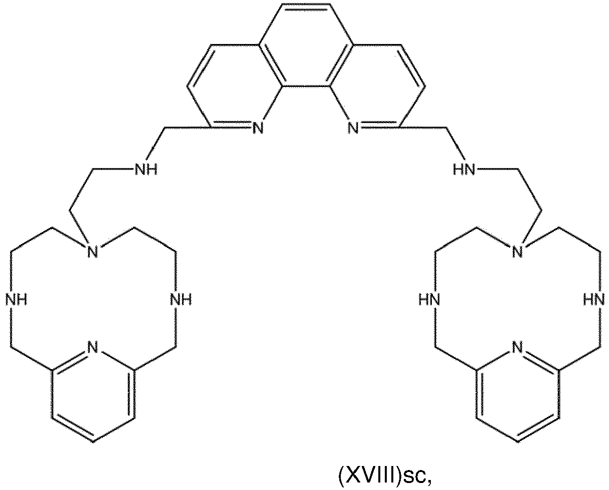 or
v. a compound selected from the group of dimethyl 1*H*- pyrazole-3,5-dicarboxylate, sodium 3,5-bis(methoxycarbonyl) pyrazolate, diethyl 1*H-*pyrazole-3,5-dicarboxylate, sodium 3,5-bis(ethoxycarbonyl) pyrazolate, dipropyl 1*H*-pyrazole-3,5-dicarboxylate, sodium 3,5-bis(propoxycarbonyl) pyrazolate, diisopropyl 1*H*-pyrazole-3,5-dicarboxylate and dibutyl 1*H*-pyrazole-3,5-dicarboxylate,
or combinations thereof.

21. The combined pharmaceutical or veterinary preparation for its use according to claim 19 or 20, wherein the compound or compounds of Formula (I) is selected from the group of compounds of formulas: 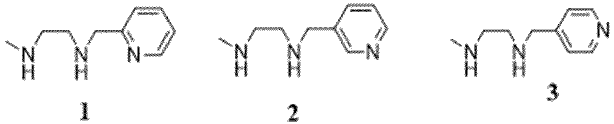 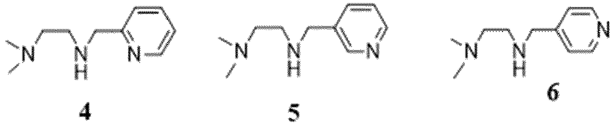 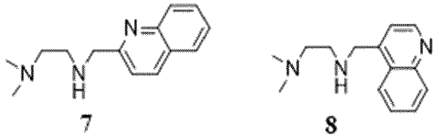 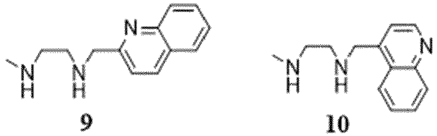 , and the pharmaceutically acceptable salts and solvates thereof,
or combinations thereof.

22. A kit-of-parts for preparing a combined pharmaceutical or veterinary preparation for use according to any one of claims 19 to 21, comprising:
a) at least one compound of Formula (I) 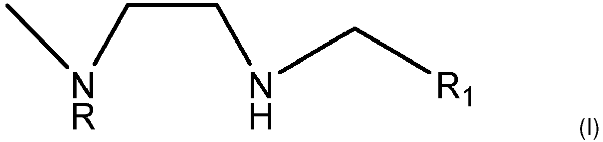 or a pharmaceutically acceptable salt or solvate thereof, wherein
R is selected from H or CH₃, and
R₁ is selected from the group of 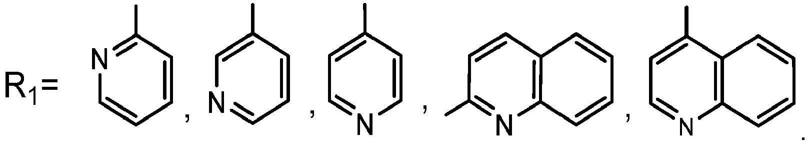
or combinations thereof,
and additionally
b) at least one antiparasitic agent with activity against a parasite of the family *Trypanosomatidae,* wherein the additional antiparasitic agent is different from the compounds defined in a).

23. The kit-of-parts for its use according to claim 22, wherein the additional antiparasitic agent is selected from the group of:
i. Meglumine antimoniate,
ii. Sodium stibogluconate,
iii. Benznidazole,
iv. a compound selected from the group of the following compounds of formulas (II)sc, (III)sc, (IV)sc, (V)sc, (VI)sc, (VII)sc, (VIII)sc, (IX)sc, (X)sc, (Xl)sc, (XII)sc, (XIII)sc, (XIV)sc, (XV)sc, (XVI)sc, (XVII)sc, and (XVIII)sc 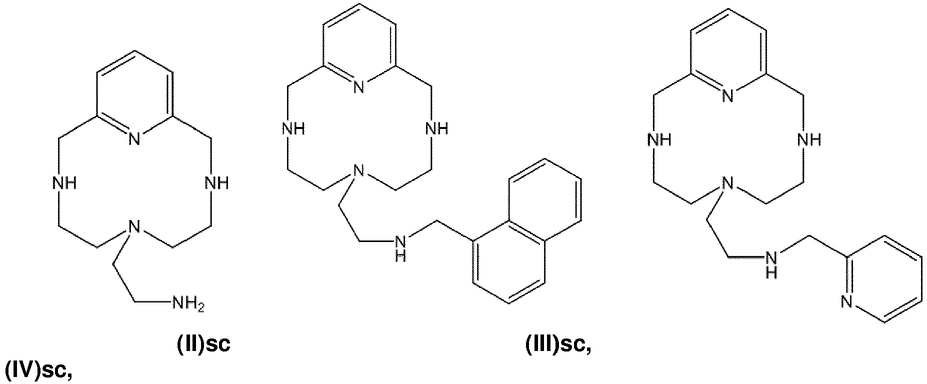 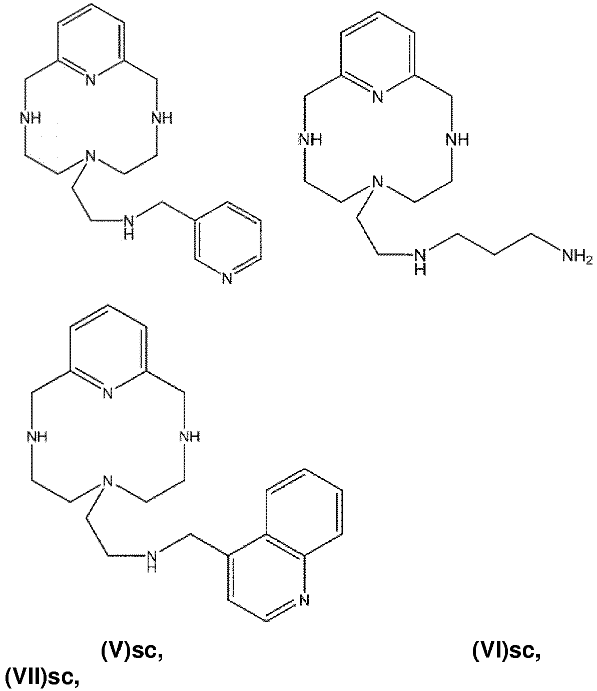 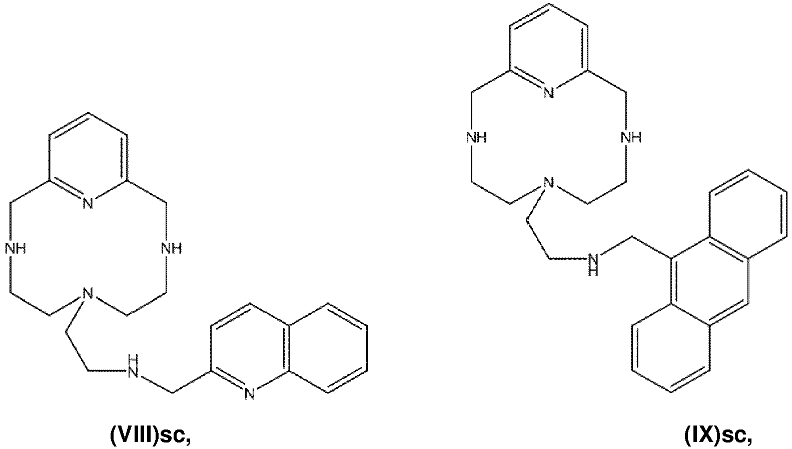 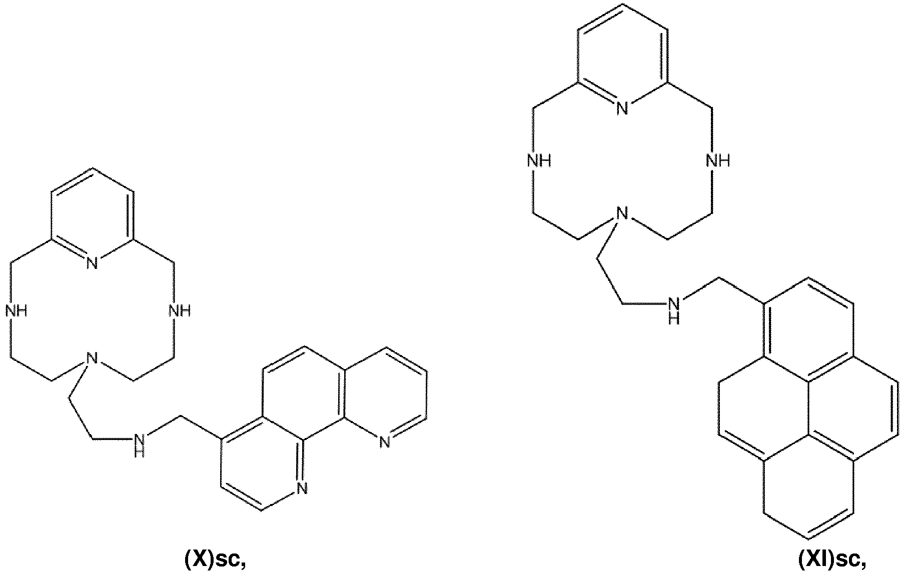 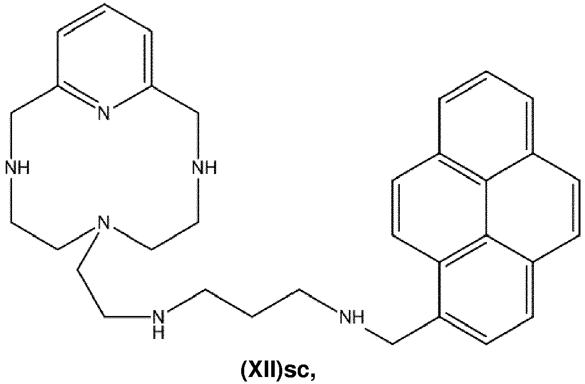 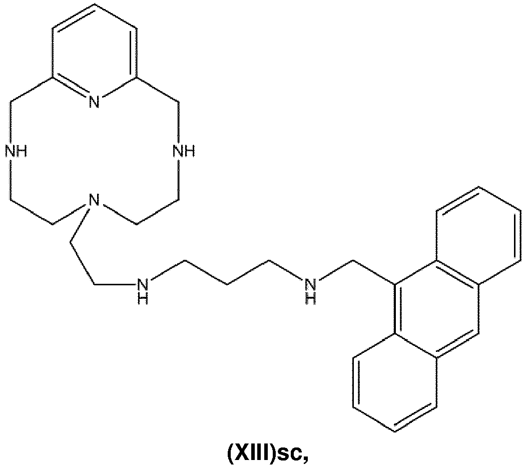 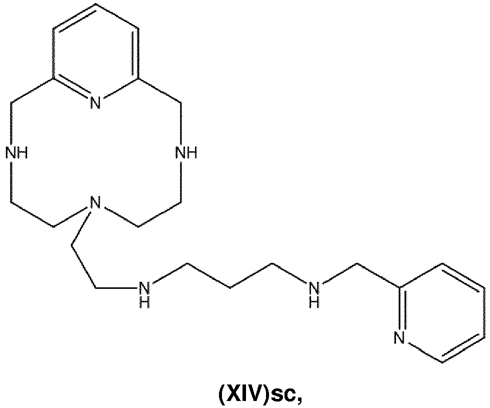 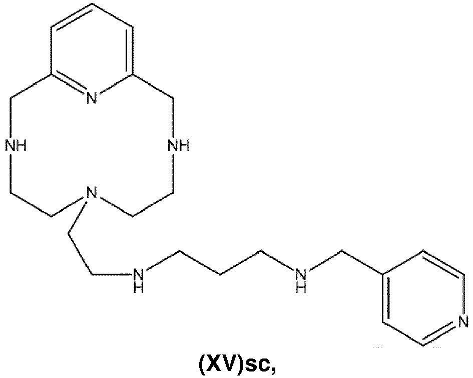 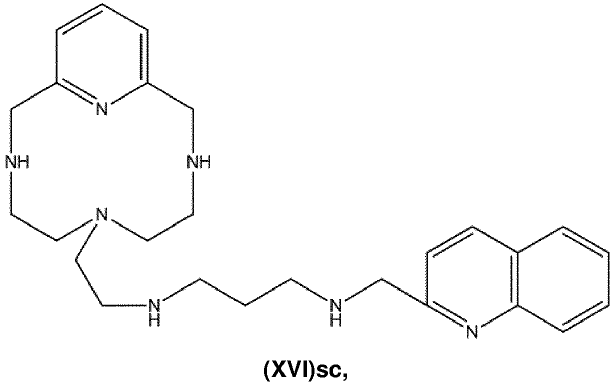 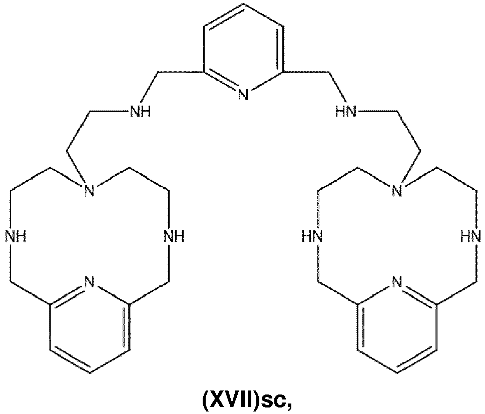 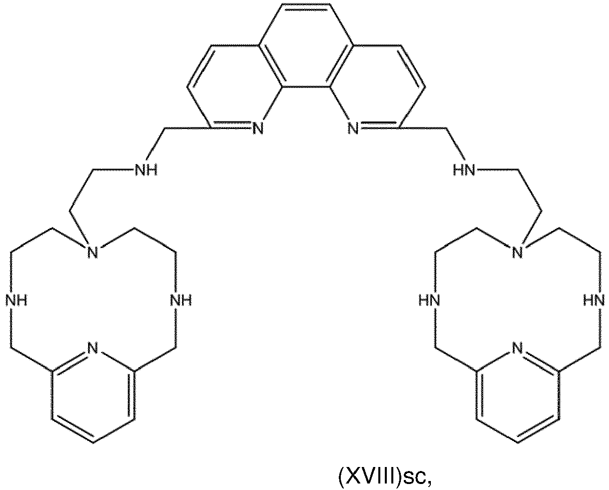 or
v. a compound selected from the group of dimethyl 1*H*- pyrazole-3,5-dicarboxylate, sodium 3,5-bis(methoxycarbonyl) pyrazolate, diethyl 1*H-*pyrazole-3,5-dicarboxylate, sodium 3,5-bis(ethoxycarbonyl) pyrazolate, dipropyl 1*H*-pyrazole-3,5-dicarboxylate, sodium 3,5-bis(propoxycarbonyl) pyrazolate, diisopropyl 1*H*-pyrazole-3,5-dicarboxylate and dibutyl 1*H*-pyrazole-3,5-dicarboxylate,
or combinations thereof.

24. The kit-of-parts for its use according to claim 22 or 23, wherein the compound or compounds of Formula (I) is selected from the group of compounds of formulas: 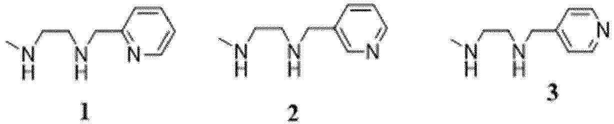 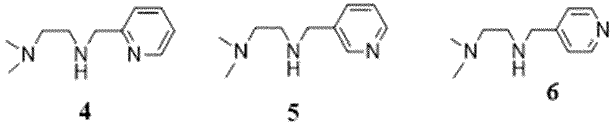 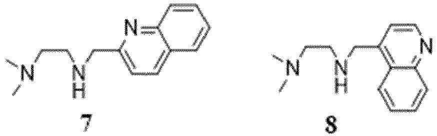 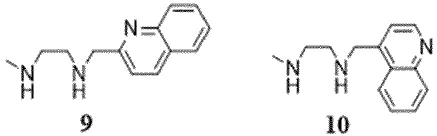 and the pharmaceutically acceptable salts and solvates thereof,
or combinations thereof.

25. A composition for its use according to any of claims 16 to 18, a pharmaceutical or veterinary preparation for its use according to any one of claims 19 to 21 or a kit-of-parts for its use according to any one of claims 22 to 24, wherein the composition, the preparation or the kit-of-parts comprises a compound selected from the group of compounds 2 (1-{3-pyridyl) -2,5-diazahexane), 4 (1-(2-pyridyl) -5-methyl-2,5-diazahexane) and 5 (1-{3-pyridyl} -5-methyl-2,5-diazahexane) and the pharmaceutically acceptable salts and solvates thereof.

26. A composition for its use according to claim 25, a pharmaceutical or veterinary preparation for its use according to claim 25 or a kit-of-parts of for its use according to claim 25, wherein the leishmaniasis is caused by a species of the genus *Leishmania* selected from *L. tropica, L. major, L. aethiopica, L. braziliensis, L. panamensis*/*L. guyanensis, L. shawi, L. peruviana, L. mexicana, L. amazonensis, L. venezuelensis, L. lainsoni, L.* naiffi, *L. linderbergi, L. infantum and L. donovani.*

27. A composition for its use according to claim 26, a pharmaceutical or veterinary preparation for its use according to claim 26 or a kit-of-parts for its use according to claim 26, wherein the leishmaniasis is caused by a species of the genus *Leishmania* selected from *L. braziliensis, L. infantum and L. donovani.*

28. A composition for its use according to claim 25, a pharmaceutical or veterinary preparation for its use according to claim 25 or a kit-of-parts for its use according to claim 25, wherein the leishmaniasis is selected from the group of cutaneous leishmaniasis, mucocutaneous leishmaniasis or visceral leishmaniasis.

29. A composition for its use according to claim 25, a pharmaceutical or veterinary preparation for its use according to claim 25 or a kit-of-parts of for its use according to claim 25, wherein the use is in the treatment of leishmaniasis in a mammal selected from the group of dog, wolf, fox, cat, horse, mule, donkey, sheep, goat, cow and human being.

30. A composition for its use according to any one of claims 25 or 26, a pharmaceutical or veterinary preparation for its use according to any one of claims 25 or 26 or a kit-of-parts for its use according to any one of claims 25 or 26, wherein the use is in the treatment of leishmaniasis in a dog.

31. A composition for its use according to any one of claims 25 to 29, a pharmaceutical or veterinary preparation for its use according to any one of claims 25 to 29 or a kit-of-parts for its use according to any one of claims 25 to 29, wherein the use is in the treatment of leishmaniasis in a human being.

32. A composition for its use according to any one of claims 16 to 18, a pharmaceutical or veterinary preparation for its use according to any one of claims 19 to 21 or a kit-of-parts for its use according to any one of claims 22 to 24, wherein its use is in the treatment of a disease caused by a parasite of the *Trypanosomatidae* family in a human being.
